# FASCICULE DE BREVET EUROPEEN

(11) **EP 3 700 619 B1**
(45) Date de publication et mention de la délivrance du brevet: **18.08.2021**
(21) Numéro de dépôt: 18792942.7
(22) Date de dépôt: 25.10.2018
(51) Int. Cl.: A61N 1/05

(54) **IMPLANT SOUPLE EN DIAMANT**
FLEXIBLES WEICHES DIAMANTIMPLANTAT
FLEXIBLE SOFT DIAMOND IMPLANT

(30) Priorité: 25.10.2017 FR 1760074
(43) Date de publication de la demande: 02.09.2020
(73) Titulaire: Chambre de Commerce et d'Industrie de Region Paris Ile de France, 93160 Noisy Le Grand (FR); Commissariat à l'Energie Atomique et aux Energies Alternatives, 75015 Paris (FR)
(72) Inventeur: SCORSONE, Emmanuel, 78114 Magny-les-Hameaux (FR); ROUSSEAU, Lionel, 94170 Le Perreux Sur Marne (FR)
(74) Mandataire: Regimbeau
(86) Numéro de dépôt international: PCT/EP2018/079353
(87) Numéro de publication internationale: WO 2019/081683

(56) Documents cités:
- EP-A1- 2 343 543
- FR-A1- 2 960 787
- FR-A1- 3 011 727
- US-A1- 2002 120 296

## Description

### Domaine de l'invention

La présente invention concerne des implants destinés à être installés sur une structure nerveuse, notamment afin de stimuler ladite structure nerveuse ou pour mesurer les signaux électriques émis par ladite structure nerveuse.

L'invention s'applique notamment aux implants rétiniens ou corticaux.

L'invention concerne également un procédé de fabrication de tels implants.

### Etat de l'art

Il est aujourd'hui possible, grâce à des implants, de restaurer certaines fonctions défaillantes chez des patients.

Il est ainsi par exemple envisageable de redonner la vue à des patients atteint de Dégénérescence Maculaire Lié à l'Age (DMLA) ou Rétinopathie Pigmentaire (RP).

Pour cela, il existe aujourd'hui en test des systèmes électroniques, ou implants dits « rétiniens », qui sont implantés au niveau de la rétine de patients atteints par ces maladies.

Ces systèmes électroniques, ou implants, qui sont implantés dans le corps du patient comprennent des microélectrodes viennent stimuler les cellules de la rétine au contact de celle-ci.

D'autres implants en cours de développement sont des implants corticaux (ou ECoG pour ElectroCorticaGraphie selon la terminologie anglo-saxonne), qui sont destinés à être directement placés sur la surface du cerveau du patient (cortex).

Cette prochaine génération d'implants doit permettre d'aller encore plus loin dans la réhabilitation fonctionnelle.

Un but est notamment de permettre aux patients dont le nerf optique est non fonctionnel de pouvoir retrouver la vue grâce à cette nouvelle génération d'implants, ce qui n'est pas le cas actuellement avec les implants rétiniens existants.

Les patients visés par la nouvelle génération d'implants sont notamment les patients atteints de la maladie du glaucome. La maladie du glaucome touchera, en 2020, 80 millions de personnes dans le monde (chiffres d'une étude du British Journal of Ophthalmology) et 9% des personnes atteintes perdront totalement la vue.

Par ailleurs, l'utilisation de ces implants n'est pas limitée aux seuls patients aveugles, mais peut également concerner les patients tétraplégiques ou paraplégiques afin de retrouver de l'autonomie.

Le but de cette nouvelle génération d'implants est de réaliser de véritables interfaces avec le cerveau pour que ces patients puissent interagir avec le monde qui les entoure. On parle alors d'Interface Cerveau Ordinateur (ou BCI pou Brain Computer Interface selon la terminologie anglo-saxonne).

Les implants ont alors pour rôle d'enregistrer ou de stimuler les activités neuronales, un décodage de ces informations étant réalisé pour commander différents systèmes (fauteuil, bras robotisé etc...).

Des applications cliniques peuvent également être envisagées pour des patients dans le coma ou touchés par le syndrome de désafférentation motrice (syndrome d'enfermement ou Lock-in syndrome selon la terminologie anglo-saxonne).

Pour ces patients dont plus aucune fonction motrice ne permet de communiquer avec le monde extérieur, ces prothèses permettraient de venir communiquer avec eux pour connaitre leur état et améliorer la connaissance sur ces pathologies pour une meilleure thérapie.

Avec le développement de ces applications, un des véritables enjeux pour cette future génération d'implants est la durée de vie desdits implants. En effet, l'implantation de ces implants dans le corps nécessite de la chirurgie très complexe. Il est préférable qu'un implant une fois implanté ne nécessite pas un remplacement fréquent.

Le but à atteindre est donc d'obtenir des implants pouvant rester implantés dans le corps d'un patient entre 5 ans et 20 ans. Or, actuellement, les certifications données pour les implants corticaux ne dépassent pas 30 jours.

Par ailleurs, les implants doivent posséder une grande souplesse afin de pouvoir épouser la forme de l'organe sur lequel lesdits implants sont installés et ne pas détériorer l'organe sur lequel lesdits implants sont installés (réaction gliale en contact des implants). La souplesse de l'implant doit être particulièrement élevée lorsque l'implant est destiné à être implanté sur le cerveau d'un patient, le cerveau étant un organe possédant une surface particulièrement accidentée.

Les implants souples actuels sont composés de deux couches de polymère qui entourent et ainsi isolent les pistes métalliques du milieu aqueux. Les couches de polymère offrent l'avantage de former une couche protectrice qui est suffisamment souple pour que l'implant puisse épouser la forme de l'organe sur lequel ledit implant est implanté.

Cependant, un problème rencontré par ces implants souples et qu'aucun polymère n'est étanche à long terme, et que les couches métalliques des implants viennent à se dégrader avec le temps, créant ainsi une dérive des implants et une modification des performances desdits implants au cours du temps suite à leur implantation.

Une première technique connue pour améliorer l'étanchéité de tels implants souples consiste à augmenter l'épaisseur des couches de polymère. Cependant, une telle augmentation de l'épaisseur des couches de polymères réduit la souplesse des implants.

Une deuxième technique connue pour améliorer l'étanchéité des implants souples consiste à réaliser des couches de barrières sur les pistes métalliques par dépôt de couches atomiques (ou ALD pour Atomic Layer Déposition selon la terminologie anglo-saxonne). Typiquement, les couches de barrières déposées peuvent être en Al₂O₃, TiO₂ ou SiC. Ces couches de barrières sont ensuite recouvertes par des couches de polymère. Cette deuxième technique est notamment décrite dans les documents « M. Saugandhika et al, Acta Biomatetialia, 960-967, 2014 », « JP Seymour et al, Biomaterials 6158,6167, 2009 X», « Lei et al, Journal of Neural Engineering. 2016 0.1088/1741-2560/13/4/046016, 2016 », et « M. Op de beeck, IMAPS Workshop Dec. 2012 ».

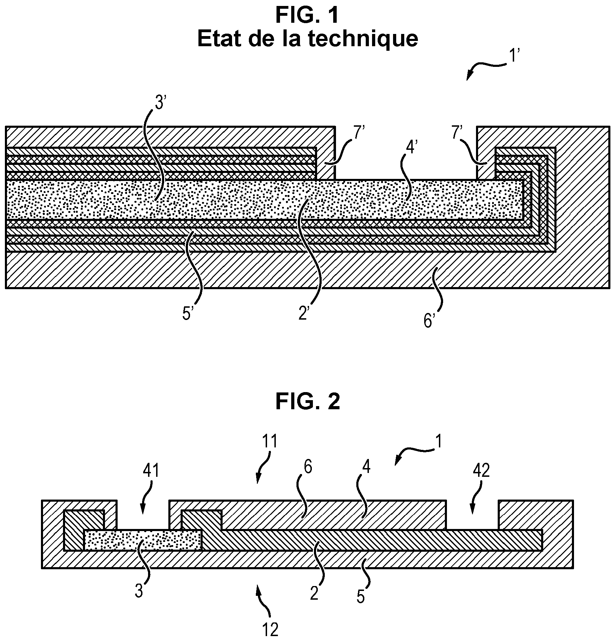

Ainsi, comme cela est représenté sur la **figure 1****,** un implant 1' selon cette deuxième technique comprend un cœur métallique 2' qui forme d'une part une piste métallique 3' et d'autre part une électrode 4'. L'électrode 4' est formée par le fait que le cœur métallique 2' n'est pas recouvert au niveau de ladite électrode 4', contrairement au niveau de la piste métallique 3'. Comme indiqué précédemment, la piste métallique 3' est entourée par des couches de barrières 5', lesdites couches de barrières 5' étant elles même entourées de couches de polymère 6'.

Cependant, comme cela est illustrée sur la **figure 1****,** cette deuxième technique rencontre un problème d'étanchéité au niveau de l'électrode 4'. En effet, l'implant 1' possède une zone de faiblesse 7' car une partie du cœur métallique 2' de l'implant 1' n'est pas recouverte par les couches de barrières 5', et uniquement par les couches de polymère 6'. Une autre solution est proposée dans le document US 2002/120296 A1.

### Présentation générale de l'invention

Un but de l'invention est de proposer une solution permettant un implant destiné à être implanté dans le corps d'un patient, typiquement directement sur le cortex du patient, qui présente à la fois une grande souplesse pour pouvoir épouser la forme de l'organe qui peut présenter des plis et sur lequel l'implant est destiné à être implanté, et une durée de vie très importante une fois implanté dans le corps du patient.

Par ailleurs, un autre but de l'invention est d'obtenir un implant qui possède un ratio signal sur bruit élevé.

A cet effet, selon un premier aspect de l'invention, il est prévu un implant souple pour la stimulation focale ou l'enregistrement électrique d'une structure nerveuse d'un organe d'intérêt, comprenant :
- une première couche en diamant isolant électriquement,
- au moins une électrode en diamant dopé conducteur électriquement en contact avec la première couche en diamant isolant électriquement,
- une couche conductrice électriquement en contact avec :
   - l'électrode, et
   - la première couche,
   pour définir une piste conductrice par électrode ;
- une seconde couche en diamant isolant électriquement au moins en contact avec la couche conductrice électriquement et une partie restante de la première couche,
agencés de façon à ce que :
- une étanchéité diamant isolant électriquement/diamant dopé conducteur électriquement est réalisée au niveau de l'électrode par reprise de croissance épitaxiale,
- la couche conductrice électriquement est encapsulée par l'électrode, par la première couche, et par la seconde couche, au niveau de l'électrode, et par la première couche, et par la seconde couche sur toute sa surface restante sauf sur une zone définissant un contact électrique par électrode,
l'implant souple disposant de deux faces :
- une face avant comportant l'une des deux couches en diamant isolant électriquement localement ouverte, permettant un accès à l'électrode et à la zone définissant un contact électrique pour l'électrode ;
- une face arrière comportant l'autre des deux couches en diamant isolant électriquement.

Cet implant est avantageusement complété par les différentes caractéristiques suivantes prises seules ou selon toutes leurs combinaisons techniquement possibles :
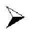 l'épaisseur totale et la géométrie de l'implant sont adaptées pour que l'implant épouse la surface de l'organe d'intérêt, tel que le cortex cortical, le cœur, ou un muscle, l'épaisseur totale de l'implant étant inférieure à 20 µm ; avantageusement elle peut être inférieure à 10 µm ;
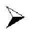 au niveau de l'électrode, la couche conductrice électriquement est encapsulée par l'électrode et la seconde couche réalisée par reprise de croissance épitaxiale à partir de l'électrode,
   l'électrode étant encapsulée, sauf sur une partie centrale de mesure, par la première couche, par la deuxième couche et par la couche conductrice électriquement ;
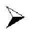 la couche conductrice électriquement est encapsulée par l'électrode et les première et seconde couches, la première couche étant réalisée par reprise de croissance épitaxiale à partir de l'électrode ;
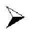 la première couche, l'électrode, et la seconde couche présentent chacune des épaisseurs inférieures à un micromètre, avantageusement 500 nm ;
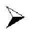 la couche conductrice électriquement présente une épaisseur inférieure à 3 µm ;
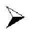 l'implant comporte au moins une couche externe en polymère biocompatible ;
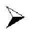 l'implant comporte une première couche externe en polymère sur la face arrière de l'implant ;
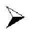 l'implant comporte une seconde couche externe en polymère sur la face avant de l'implant sauf au niveau d'au moins une partie de mesure de l'électrode et au niveau d'une zone de contact électrique,
   l'implant étant ainsi complètement encapsulé par du polymère, sauf au niveau de la partie de mesure de l'électrode et au niveau de la zone de contact électrique ;
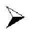 la (ou les) couche(s) externe(s) en polymère présente(nt) une épaisseur comprise entre 2 et 10 µm ;
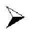 le polymère est un polymère biodégradable ;
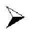 l'électrode en diamant dopé est nanostructurée ;
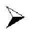 le diamant des éléments suivants : électrode, première couche, seconde couche, et couche conductrice électriquement quand elle est en diamant,
   n'est pas ultra-nanocristallin (UNCD) ;
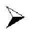 le diamant pour l'électrode est dopé au bore ou au phosphore ;
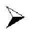 le rapport de la longueur entre l'ouverture située au niveau de l'électrode et l'ouverture située au niveau de la piste, sur l'épaisseur de l'implant, est supérieur à 100

L'invention propose également un procédé de fabrication d'au moins un implant souple pour la stimulation focale ou l'enregistrement électrique d'une structure nerveuse d'un organe d'intérêt, du type précité, ledit implant étant réalisé par les étapes suivantes à partir d'un substrat S :
- (E1) réalisation d'une première couche en diamant isolant électriquement et d'au moins une électrode en diamant dopé conducteur électriquement par croissance épitaxiale, l'électrode et la première couche étant déposées l'une sur l'autre par reprise de croissance épitaxiale à l'issue de cette étape (E1) ;
- (E2) dépôt d'une couche conductrice électriquement en appui sur au moins une partie de l'électrode et au moins une partie donnée de la première couche, pour définir une piste conductrice par électrode ;
- (E3) réalisation d'une seconde couche en appui sur au moins une partie de la couche conductrice électriquement, et au moins sur une partie restante de la première couche (qui a une surface libre), par croissance épitaxiale ;
les étapes de procédé sont réalisées pour que :
- une étanchéité diamant isolant électriquement/diamant dopé conducteur électriquement est réalisée au niveau de l'électrode par reprise de croissance épitaxiale,
- la couche conductrice électriquement est encapsulée par l'électrode, par la première couche, et par la seconde couche, au niveau de l'électrode et sur toute sa surface restante sauf sur une zone définissant un contact électrique .

Le procédé est avantageusement complété par les différentes caractéristiques suivantes prises seules ou selon toutes leurs combinaisons techniquement possibles :
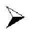 l'épaisseur totale de l'implant souple est inférieure à 20 µm ;
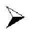 le procédé comprend les étapes suivantes :
   - dans l'étape (E1), réalisation par croissance épitaxiale de la première couche en diamant isolant électriquement sur le substrat S, puis réalisation par reprise de croissance épitaxiale d'au moins une électrode en diamant dopé conducteur électriquement en appui sur la première couche,
   - dans l'étape (E2), dépôt de la couche conductrice électriquement sur un contour extérieur de l'électrode et au moins sur une partie donnée de la première couche,
   - dans l'étape (E3), la croissance épitaxiale de la deuxième couche est réalisée sur :
      la couche conductrice électriquement sauf sur une zone définissant un contact électrique par piste conductrice,
      ainsi que sur l'électrode par reprise de croissance épitaxiale, en laissant à l'air libre une partie centrale de mesure de l'électrode,
      cette reprise de croissance diamant isolant électriquement sur diamant dopé conducteur électriquement permet de garantir l'étanchéité de l'implant au niveau de l'électrode ;
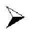 le procédé comprend les étapes suivantes :
   - dans l'étape (E1), réalisation par croissance épitaxiale d'au moins une électrode en diamant dopé conducteur électriquement sur le substrat S, puis réalisation de la première couche en diamant sur le conteur extérieur de l'électrode par reprise de croissance épitaxiale et sur une partie du substrat S par croissance épitaxiale sauf dans une zone donnée qui définit un contact électrique,
   - dans l'étape (E2), dépôt de la couche conductrice sur au moins une partie centrale de l'électrode, sur au moins une partie donnée de la première couche pour réaliser une piste conductrice par électrode, et sur la zone donnée de contact électrique pour réaliser un contact électrique par piste conductrice ;
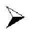 le procédé comprend l'étape supplémentaire suivante (E4) de dépôt d'au moins une couche externe en polymère, par exemple sur la face arrière de l'implant ;
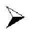 le procédé comprend les étapes supplémentaires suivantes :
   - (E41) dépôt d'une couche externe en polymère sur la face avant de l'implant, où se situent l'électrode et le contact électrique associé, ainsi que sur le substrat S ;
   - (E42) ouverture de la couche externe en polymère au niveau du contact électrique et de l'électrode et dépôt d'une résine de protection ;
   - (E43) gravure du substrat jusqu'à découvrir la face arrière de l'implant ;
   - (E44) dépôt d'une autre couche externe en polymère sur la face arrière de l'implant élimination de la résine de protection et découpe de l'implant pour lui donner sa forme finale ;
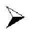 l'électrode est nanostructurée de la façon suivante :
   - dépôt sur l'électrode d'un matériau formant un motif 3D nanostructuré ;
   - dépôt d'une couche en diamant dopé conducteur électriquement par-dessus le matériau formant le motif nanostructuré.

### Présentation des figures

D'autres caractéristiques, buts et avantages de l'invention apparaitront à la lecture de la description ci-après de différents modes de réalisation représentés dans les dessins suivants qui ne sont pas à l'échelle réelle, l'implant étant bien plus long qu'épais:
- la figure 1 représente un implant selon l'état de la technique
- la figure 2 représente une vue en coupe simplifiée d'implant selon un premier mode de réalisation de l'invention ;
- la figure 3a représente un implant selon le premier mode de réalisation comprenant une couche de polymère sur sa face arrière ;
- la figure 3b représente un implant selon le premier mode de réalisation comprenant une couche de polymère sur sa face arrière et une couche de polymère sur sa face avant ;
- la figure 4 représente une vue en coupe simplifiée d'implant selon un deuxième mode de réalisation de l'invention ;
- la figure 5 représente un implant selon le deuxième mode de réalisation comprenant une couche de polymère sur sa face arrière ;
- la figure 6 représente schématiquement les étapes d'un procédé de fabrication de l'implant ;
- la figure 7 représente schématiquement les étapes d'une variante du procédé de fabrication pour le dépôt de couches de polymère sur la face avant et sur la face arrière de l'implant ;
- les figures 8a, 8b, 8c, 8d, 8e, 8f, 8g, et 8h représentent les différentes états de l'implant lors des différentes étapes du procédé de fabrication selon une première mise en œuvre possible pour la réalisation d'un implant selon le premier mode de réalisation de l'invention, l'implant étant ici uniquement en diamant + métal (sans enrobage polymère) et autoporté;
- les figures 9a et 9b représentent différents états de l'implant lors des étapes de dépôt d'une couche en polymère uniquement sur la face arrière de l'implant selon une variante possible de la première mise en œuvre du procédé de fabrication ;
- les figures 10a, 10b, 10c, 10d et 10e représentent différents états de l'implant lors des étapes de dépôt d'une couche en polymère sur la face avant et la face arrière de l'implant selon une variante possible de la première mise en œuvre du procédé de fabrication ;
- les figures 11a, 11b, 11c, 11d, 11e, 11f, 11g, et 11h représentent les différentes états de l'implant lors des différentes étapes du procédé de fabrication selon une seconde mise en œuvre possible pour la réalisation d'un implant selon le deuxième mode de réalisation de l'invention ;
- la figure 12 représente un état intermédiaire de l'implant lors d'une étape de dépôt d'une couche de polymère sur face arrière de l'implant selon une variante de la seconde mise en œuvre possible du procédé de fabrication ;
- les figures 13a-13g représentent des vues de dessus des différents états de l'implant selon le premier mode de réalisation lors de sa fabrication ;
- la figure 14 représente un implant selon l'invention disposé sur un gant en latex dont la surface simule la surface d'une structure nerveuse sur laquelle l'implant est destiné à être implanté.

### Description de l'invention

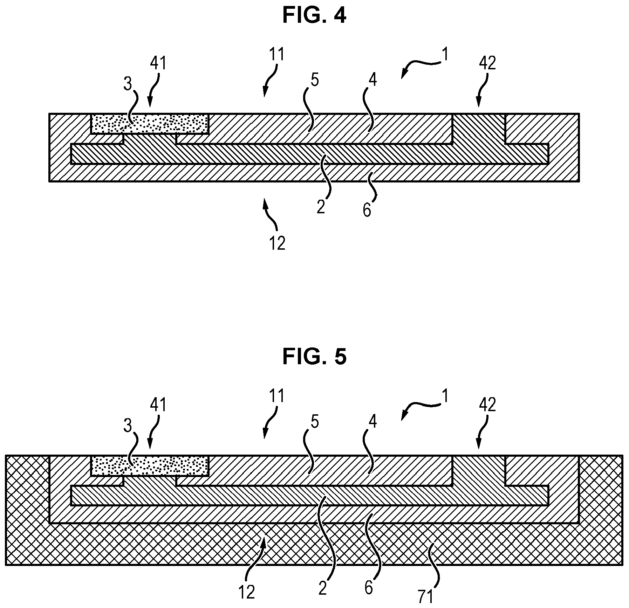

Comme représenté sur la **figure 2****,** qui représente un implant souple 1 selon un premier mode de réalisation, et sur la **figure 4****,** qui représente l'implant souple 1 selon un deuxième mode de réalisation, ledit implant souple 1 comprend :
- une couche conductrice électriquement 2, qui peut être en métal ou en diamant dopé conducteur électriquement ;
- une ou des électrodes 3, en diamant dopé conducteur électriquement, par exemple dopé au bore ou au phosphore, chacune en contact avec la couche conductrice électriquement 2, ladite couche conductrice électriquement 2 présentant ainsi une piste électrique par électrode 3 de l'implant ;
- un revêtement protecteur 4 en diamant isolant électriquement, typiquement du diamant intrinsèque (c'est-à-dire non-dopé), qui encapsule/entoure la couche conductrice électriquement 2 et la ou les électrodes 3 sauf au niveau des zones 41 et 42 de chaque électrode.

Le revêtement protecteur 4 comprend une première ouverture 41 au niveau de chaque électrode 3, afin de permettre à chaque électrode 3 d'être couplée avec la structure nerveuse désirée. Le revêtement protecteur 4 comprend également une deuxième ouverture 42 au niveau de la couche conductrice électriquement 2 afin de former une zone de contact électrique pour une électrode, et permettre ainsi de connecter l'implant souple 1 à un composant électronique pour contrôler le couplage électrique entre l'électrode 3 et la structure nerveuse.

L'implant souple 1 comprend une face avant 11 sur laquelle sont situées la première ouverture 41 et la deuxième ouverture 42, et une face arrière 12 qui est opposée à ladite face avant 11.

Le fait que le revêtement protecteur 4 soit en diamant non conducteur électriquement et que l'électrode 3 soit en diamant dopé conducteur électriquement permet d'assurer une protection à long terme de la couche conductrice électriquement 2 qui se retrouve complètement encapsulée par du diamant (sauf au niveau de la deuxième ouverture 42 qui n'est pas au contact de tissu biologique), le diamant étant parfaitement étanche car la densité du diamant empêche les particules de diffuser au travers. En outre, le diamant offre l'avantage d'être biocompatible, et peut donc être implanté dans le corps humain.

De plus, l'étanchéité de l'implant souple 1 est assurée par le fait que les contacts entre l'électrode 3 et le revêtement protecteur 4 sont des zones de reprise de croissance épitaxiale de l'électrode 3 sur le revêtement protecteur 4, et du revêtement protecteur 4 sur l'électrode 3. Une telle reprise de croissance assure qu'il n'y a pas de discontinuité entre l'électrode 3 et le revêtement protecteur 4, garantissant ainsi l'étanchéité de l'implant 1.

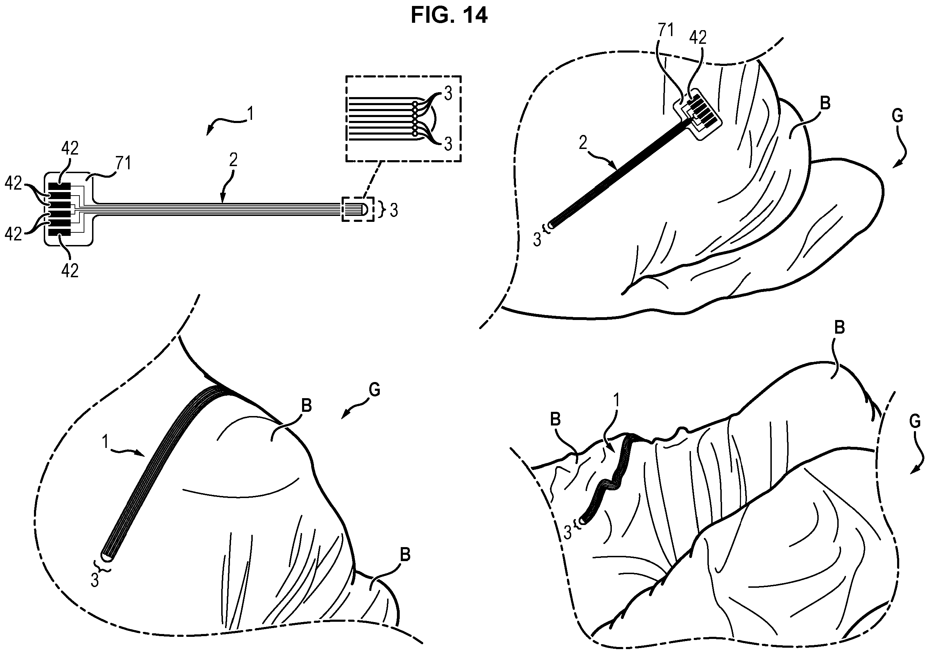

Par ailleurs, comme cela est illustré sur la figure 14, l'implant 1 possède une grande souplesse, permettant ainsi audit implant 1 de suivre les différentes aspérités de la surface sur laquelle ledit implant est positionné. Une telle souplesse avec un implant comprenant un revêtement protecteur 4 en diamant est surprenante car le diamant est un matériau rigide. La souplesse de l'implant est notamment due à son épaisseur très faible.

A des fins de simplification, l'implant 1 est décrit avec une électrode 3, mais l'implant 1 comprend de préférence une pluralité d'électrodes 3 en contact avec la couche conductrice électriquement 2, ladite couche conductrice électriquement 2 formant une piste électrique pour chaque électrode 3 de l'implant.

Comme illustré sur la **figure 2****,** l'implant souple 1 selon le premier mode de réalisation comprend une première couche 5 en diamant isolant électriquement qui est en contact avec l'électrode 3 en diamant et la couche conductrice électriquement 2, la première couche 5 formant une première partie du revêtement protecteur 4. L'implant 1 comprend également une seconde couche 6 en diamant isolant électriquement qui est en contact avec l'électrode 3 et la couche conductrice électriquement 2, la seconde couche 6 formant une seconde partie du revêtement protecteur 4. La première couche 5 et la seconde couche 6 sont également en contact partout où il n'y a pas les électrodes 3 et la couche conductrice électriquement 2. Les termes première couche 5 et seconde couche 6 font ici référence à l'ordre dans lequel les couches 5 et 6 sont fabriquées.

La seconde couche 6 est située sur la face avant 11 de l'implant 1 et comprend ainsi la première ouverture 41 et la deuxième ouverture 42, la première couche 5 étant située sur la face arrière 12 dudit implant 1.

La couche conductrice électriquement 2 est encapsulée (limitée ou située enfermée, enveloppée, entourée ou enrobée) par l'électrode 3, par la première couche 5, et par la seconde couche 6, au niveau de l'électrode 3, et par la première couche 5 et la seconde couche 6 sur toute sa surface restante (longueur, largeur et épaisseur) sauf sur la zone définissant le contact électrique à cause de la seconde ouverture 42. De plus, au niveau de l'électrode 3, la couche conductrice électriquement 2 est encapsulée par l'électrode 3 et la seconde couche 6.

L'électrode 3 est réalisée par reprise de croissance à partir de la première couche 5, et la seconde couche 6 réalisée par reprise de croissance à partir de l'électrode 3, assurant ainsi l'étanchéité de l'implant souple 1.

L'électrode 3 est encapsulée (ou limitée ou située enfermée, enveloppée, entourée ou enrobée) par la première couche 5, par la deuxième couche 6, et par la couche conductrice électriquement 2, sauf sur une partie centrale de mesure correspondant à la première ouverture 41.

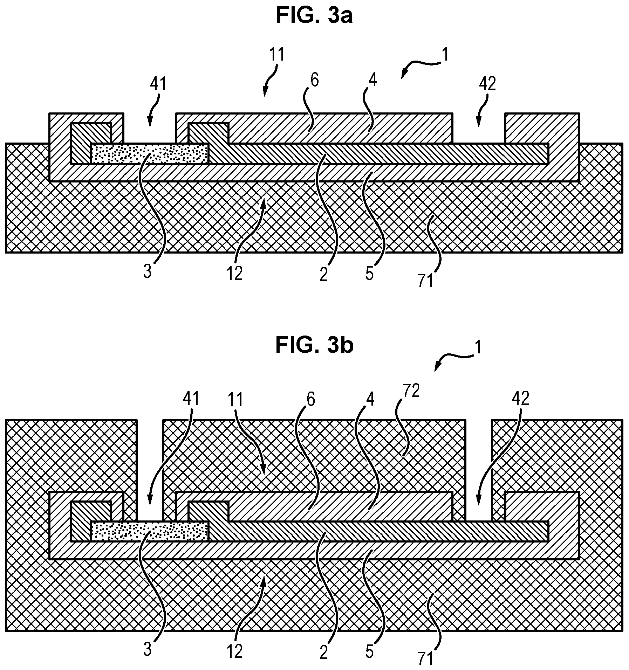

Comme cela est illustré sur les **figures 3a et 3b****,** l'implant souple 1 peut comprendre une couche de polymère, de préférence du polymère biocompatible, qui est située sur la face arrière 12 et/ou sur la face avant 11 de l'implant souple 1.

Cette couche de polymère permet de faciliter la manipulation de l'implant souple 1, notamment par le médecin lors de l'implantation de l'implant 1, la souplesse et la finesse de l'implant 1 pouvant rendre l'implant 1 difficilement manipulable.

Le polymère peut être biodégradable afin d'être éliminé après implantation de l'implant souple 1 dans le corps du patient, le polymère n'étant utile que lors de l'implantation de l'implant souple 1.

Dans la variante illustrée sur la **figure 3a****,** l'implant 1 comprend une première couche externe en polymère 71 qui est située sur la face arrière 12 dudit implant 1.

Dans la variante illustrée sur la **figure 3b****,** en plus d'avoir la première couche externe en polymère 71 sur sa face arrière 12, l'implant 1 comprend une seconde couche externe en polymère 72 sur sa face avant 11. La seconde couche externe en polymère 72 comprend des ouvertures réalisées en regard de la première ouverture 41 et de la deuxième ouverture 42 afin de garder l'accès à l'électrode 3 et à la zone de contact électrique. Ainsi, dans cette variante l'implant est complètement encapsulé par du polymère, sauf au niveau de la partie de mesure de l'électrode 3 et de la zone de contact électrique.

La ou les couches de polymères déposées sur l'implant 1 ont de préférence une épaisseur comprise entre 2 et 10 µm, afin de faciliter la manipulation de l'implant 1 tout en gardant un implant 1 avec une souplesse satisfaisante.

Comme illustré sur la **figure 4****,** l'implant 1 selon le deuxième mode de réalisation comprend également une première couche 5 en diamant isolant électriquement qui est en contact avec l'électrode 3 et la couche conductrice électriquement 2, la première couche 5 formant une première partie du revêtement protecteur 4. L'implant 1 comprend également une seconde couche 6 en diamant isolant électriquement qui est en contact avec l'électrode 3 et la couche conductrice électriquement 2, la seconde couche 6 forme une seconde partie du revêtement protecteur 4. La première couche 5 et la seconde couche 6 sont également en contact l'une avec l'autre partout où il n'y a pas les électrodes 3 et la couche conductrice électriquement 2. Là encore, les termes première couche 5 et seconde couche 6 font ici référence à l'ordre dans lequel les couches 5 et 6 sont fabriquées.

La première couche 5 est située sur la face avant 11 de l'implant 1 et comprend ainsi la première ouverture 41 et la deuxième ouverture 42, tandis que la deuxième couche 6 est située sur la face arrière 12.

Dans ce mode de réalisation, la couche conductrice électriquement 2 est encapsulée par l'électrode 3 et par les première et seconde couches en diamant 5 et 6 au niveau de l'électrode 3 et sur toute sa surface restante (longueur, largeur et épaisseur) par les première et seconde couches en diamant 5 et 6, sauf sur la zone définissant le contact électrique. De plus, au niveau de l'électrode 3, la couche conductrice électriquement 2 est encapsulée par l'électrode 3 et les premières et secondes couches en diamant 5 et 6, la première couche 5 étant réalisée par reprise de croissance à partir de l'électrode 3.

Dans le deuxième mode de réalisation, l'électrode 3 possède sa face de couplage, la face destinée à être mise en regard de la structure nerveuse, qui affleure de la première couche 5 sur toute sa surface.

Comme cela est illustré sur la **figure 5****,** l'implant 1 selon le deuxième mode de réalisation peut également comprendre une couche de polymère, de préférence du polymère biocompatible, qui est située sur la face arrière 12de l'implant souple 1.

Là encore, le polymère peut être biodégradable afin d'être éliminé après implantation de l'implant 1 dans le corps du patient, le polymère n'étant utile que lors de l'implantation de l'implant 1.

Dans la variante illustrée sur la **figure 5** qui est une variante similaire à celle illustrée sur la **figure 3a****,** l'implant 1 comprend la première couche externe en polymère 71 qui est située sur la face arrière 12 dudit implant 1.

La ou les couches de polymères déposées sur l'implant 1 ont de préférence une épaisseur comprise entre 2 et 10 µm, afin de faciliter la manipulation de l'implant 1 tout en gardant un implant 1 avec une souplesse satisfaisante.

Pour les deux modes de réalisation, l'électrode 3, la première couche 5 et la seconde couche 6 présentent chacune une épaisseur inférieure à 500 nm et maximum 1 µm, permettant ainsi à l'implant 1 d'avoir une souplesse très importante, malgré le fait que l'électrode 3, la première couche 5 et la seconde couche 6 sont en diamant.

Par ailleurs, pour les deux modes de réalisation, la couche conductrice électriquement 2 présente une épaisseur inférieure à 3 µm, afin d'assurer la souplesse de l'implant.

De plus, pour les deux modes de réalisation, l'électrode 3 est de préférence nanostructurée afin d'augmenter le ratio signal sur bruit. La face de l'électrode 3 destinée à être en regard de la structure nerveuse n'est pas lisse, mais possède un relief nanométrique, de sorte à augmenter la surface de cette face de l'électrode 3 située en regard de la structure nerveuse.

La nano structuration de l'électrode 3, ou autrement dit le fait de donner un relief de dimension nanométrique à une face de l'électrode 3, peut être réalisée de la manière suivante :
- dépôt d'un matériau formant un motif 3D nanostructuré sur une face de l'électrode 3 ;
- dépôt d'une couche en diamant dopé conducteur électriquement par-dessus le matériau formant le motif 3D, de manière à reformer l'électrode 3 avec une face qui suit le motif 3D du matériau, et ainsi est nanostructurée. Le diamant dopé peut par exemple être déposé par dépôt chimique en phase gazeuse (ou CVD), le matériau comprenant le motif 3D devant dans ce cas résister à la haute température nécessaire à la croissance d'une couche en diamant par dépôt CVD.

Selon un premier exemple possible pour réaliser l'électrode 3 avec une face nanostructurée, après avoir fait croitre le diamant dopé pour former l'électrode 3, une mousse en polymère, comme par exemple du polypyrrole, est déposée sur la face de l'électrode 3 destinée à former la face de couplage avec la structure nerveuse. Ensuite, une couche de diamant dopé est formée sur la mousse en polymère, cette couche de diamant dopé est nanostructurée car elle épouse la forme très accidentée de la mousse en polymère, et forme un motif 3D (pour 3 dimensions).

Selon un autre exemple possible pour réaliser l'électrode 3 avec une face nanostructurée, après avoir fait croitre le diamant dopé pour former l'électrode 3, des nanotubes de carbone sont déposés sur la face de l'électrode 3 destinée à former la face de couplage avec la structure nerveuse. Ensuite, une couche de diamant dopé est formée sur les nanotubes de carbone, cette couche de diamant dopé est nanostructurée car elle épouse le relief très accidenté de dimension nanométrique créé par les nanotubes de carbone, et forme un motif 3D (pour 3 dimensions).

L'électrode 3 peut bien sûr être nanostructurée par dépôt d'autres matériaux conducteurs présentant également une grande porosité et une bonne tenue aux conditions de croissance du diamant de synthèse.

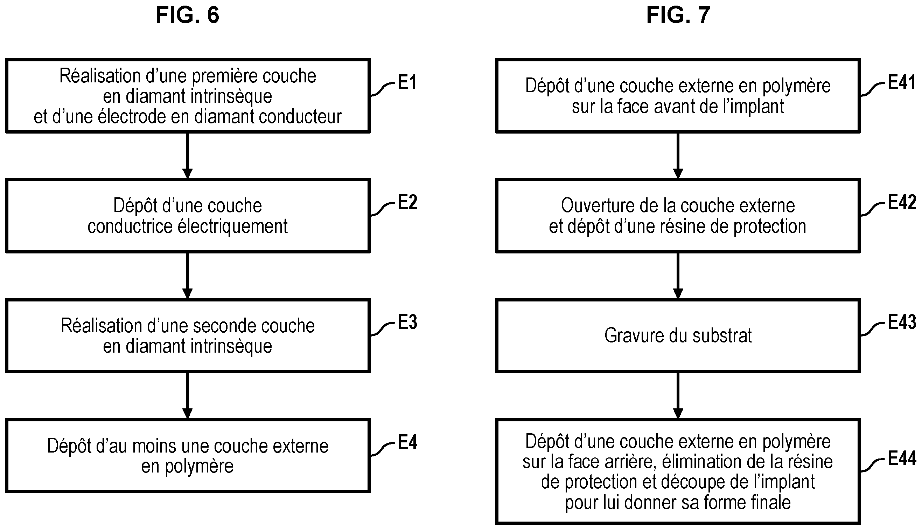

L'implant souple 1 selon l'un quelconque des modes de réalisation présentés précédemment peut être obtenu par le procédé de fabrication illustré à la figure 6 et qui comprend les étapes suivantes :
- E1 : réalisation d'une première couche 5 en diamant isolant électriquement et d'au moins une électrode 3 en diamant dopé conducteur électriquement par croissance épitaxiale, l'électrode 3 et la première couche 5 étant déposées l'une sur l'autre par reprise de croissance épitaxiale à l'issue de cette étape E1 ;
- E2 : dépôt d'une couche conductrice électriquement 2 en appui sur au moins une partie de l'électrode 3 et au moins une partie de la première couche 5, définissant ainsi une piste conductrice pour l'électrode 3 ;
- E3 : réalisation d'une seconde couche 6 en diamant isolant électriquement en appui sur au moins une partie de la couche conductrice électriquement 2, et au moins sur une partie de la partie restante de la première couche 5, par croissance épitaxiale.

Les étapes de procédé sont réalisées pour que :
- une étanchéité diamant isolant électriquement/diamant dopé conducteur électriquement est réalisée au niveau de l'électrode par reprise de croissance épitaxiale,
- la couche conductrice électriquement 2 est encapsulée par l'électrode 3 en diamant dopé conducteur électriquement, par la première couche 5 en diamant isolant électriquement, et par la seconde couche 6 en diamant isolant électriquement, au niveau de l'électrode 3 et sur toute sa surface restante (longueur, largeur et épaisseur), sauf sur une zone définissant un contact électrique (cette zone correspondant à la deuxième ouverture 42).

Le procédé peut également comprendre une étape E4 de dépôt d'au moins une couche externe en polymère, par exemple sur la face arrière 12 de l'implant 1 ou la face avant 11 de l'implant 1.

Selon une variante possible de l'étape E4 illustrée sur la **figure 7** dans laquelle deux couches de polymère sont déposées sur d'une part la face avant 11 de l'implant 1 et d'autre part la face arrière 12 de l'implant souple 1, l'étape E4 comprend les étapes suivantes :
- E41 : dépôt d'une couche en polymère sur la face avant 11 de l'implant souple 1, où se situent l'électrode 3 et le contact électrique associé formé par la couche conductrice électriquement 2, ainsi que sur le substrat sur lequel l'implant est fabriqué. La couche en polymère déposée dans cette étape est la seconde couche externe en polymère 72.
- E42 : ouverture de la couche en polymère au niveau du contact électrique et de l'électrode 3, ouvrant ainsi la couche en polymère au niveau de la première ouverture 41 et de la deuxième ouverture 42, et dépôt d'une résine de protection.
- E43 : gravure du substrat jusqu'à découvrir la face arrière 12 de l'implant souple 1.
- E44 : dépôt d'une autre couche en polymère sur la face arrière 12 de l'implant souple 1. La couche en polymère déposée dans cette étape correspond à la première couche externe en polymère 71 ; élimination de la résine de protection et découpe de l'implant pour lui donner sa forme finale.

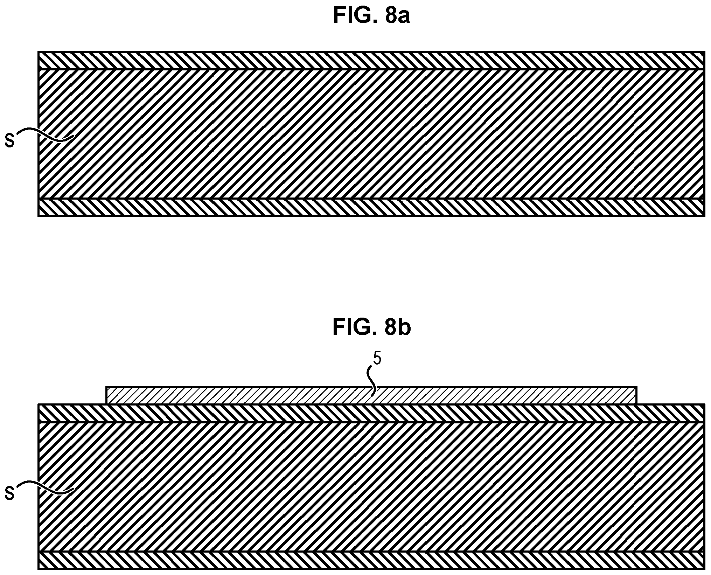

Une première mise en œuvre possible du procédé de fabrication permettant de fabriquer un implant 1 selon le premier mode de réalisation est illustrée sur les **figures 8a - 8h****.**

Comme visible sur la **figure 8a****,** un substrat S est fourni au début du procédé de fabrication afin de réaliser la succession d'étapes de dépôt des différentes couches formant l'implant souple 1.

Dans la première mise en œuvre du procédé, comme illustrée sur la **figure 8b****,** la formation de la première couche 5 en diamant isolant électriquement est réalisée en premier. La première couche 5 en diamant isolant électriquement est réalisée en déposant une couche de nanoparticules de diamant sur le substrat qui servent de sites de nucléation pour la croissance épitaxiale du diamant. La croissance de la première couche 5 est ensuite réalisée par dépôt chimique en phase vapeur (ou CVD). La croissance pour le diamant étant faite à hautes températures, il est préférable que le substrat S soit en silicium, ou en quartz, plutôt qu'en polymère.

Afin de déterminer la zone sur laquelle la première couche 5 est formée, plusieurs solutions sont possibles. Il est par exemple possible de déposer les nanoparticules de diamant sur l'ensemble du substrat S, puis de retirer les nanoparticules de diamant par gravure dans les zones sur lesquelles il n'est pas souhaité que la première couche 5 soit formée, la zone d'intérêt étant quant à elle protégée de la gravure par un masque qui protège les nanoparticules de diamant sur cette zone d'intérêt. Il est également possible de déposer les nanoparticules de diamant uniquement sur la zone d'intérêt en recouvrant les zones sur lesquelles il n'est pas souhaitée que la première couche 5 soit formée par un masque lors du dépôt des nanoparticules de diamant.

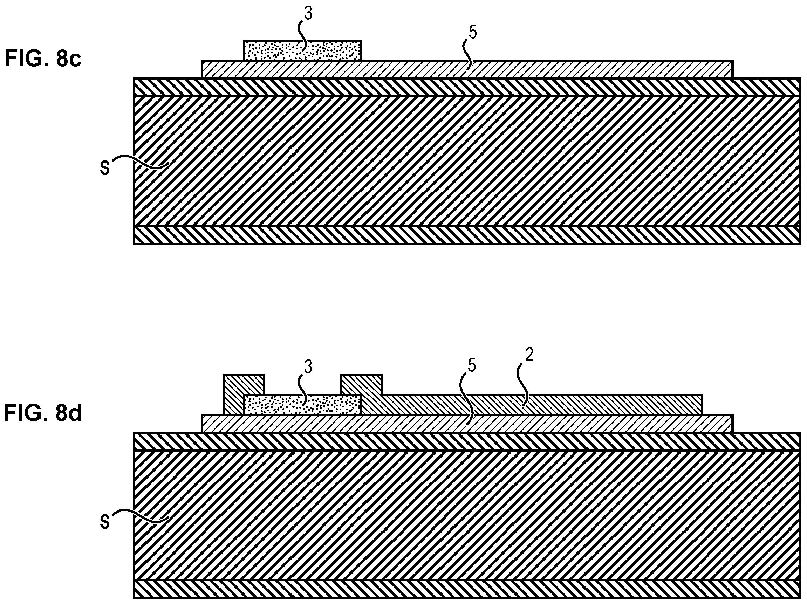

Ensuite, comme illustré sur la **figure 8c****,** l'électrode 3 est formée par la création d'une couche en diamant dopé conducteur électriquement sur la première couche 5 en diamant isolant électriquement. L'électrode 3 est formée par reprise de croissance épitaxiale à partir de la première couche 5, réalisant ainsi une continuité de matière entre les deux couches en diamant.

Afin de déterminer la zone sur laquelle l'électrode 3 est formée, plusieurs solutions sont possibles. Il est par exemple possible de déposer les nanoparticules de diamant sur l'ensemble de la couche 5 et du substrat S, puis de retirer les nanoparticules de diamant par gravure dans les zones sur lesquelles il n'est pas souhaitée que l'électrode 3 soit formée, la zone d'intérêt étant quant à elle protégée de la gravure par un masque qui protège les nanoparticules de diamant sur cette zone d'intérêt. Il est également possible de déposer les nanoparticules de diamant uniquement sur la zone d'intérêt en recouvrant les zones sur lesquelles il n'est pas souhaité que l'électrode 3 soit formée par un masque lors du dépôt des nanoparticules de diamant.

L'électrode 3 peut être nanostructurée au cours de cette étape, par exemple par une des méthodes décrites précédemment.

Comme illustrée par la **figure 8d****,** la couche conductrice électriquement 2 est ensuite formée d'une part sur la première couche 5, et d'autre part sur le contour extérieur de l'électrode 3. La couche conductrice électriquement 2 est formée par le dépôt d'une couche métallique, par exemple par dépôt chimique en phase vapeur. La couche conductrice électriquement 2 peut également être formée par le dépôt d'une couche en diamant dopé conducteur électriquement. La couche conductrice électriquement 2 est formée de manière à former une piste électrique pour l'électrode 3, et ainsi relier ladite électrode 3 à un composant électrique. Là encore, le contrôle de la forme de la couche conductrice électriquement 2 est réalisé par l'utilisation d'un masque.

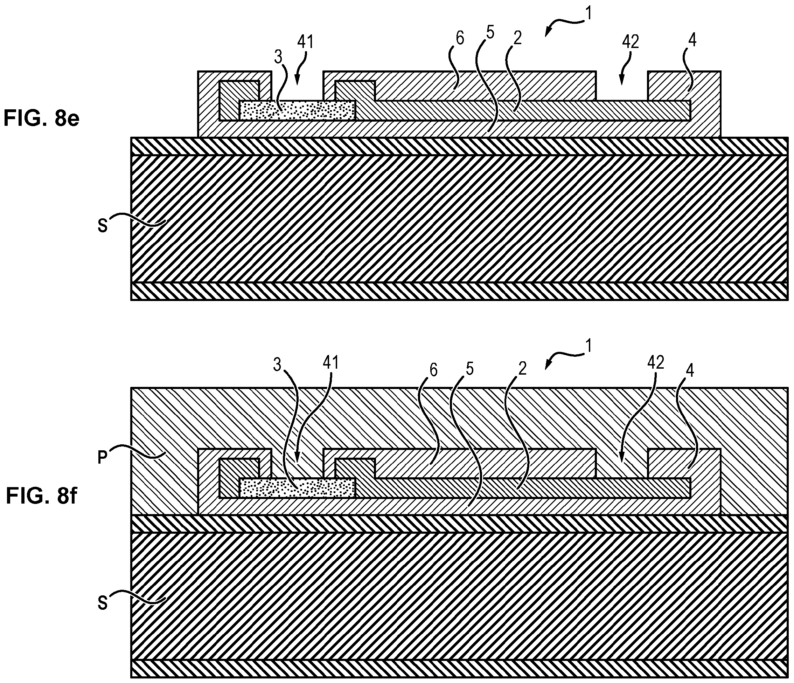

Comme illustré sur la **figure 8e****,** la seconde couche 6 en diamant isolant électriquement est ensuite formée par-dessus l'ensemble réalisé lors les étapes précédentes, finalisant ainsi la formation du revêtement protecteur 4 autour de la couche conductrice électriquement 2. La seconde couche 6 n'est toutefois pas déposée sur la zone centrale de l'électrode 3, formant ainsi la première ouverture 41, ni sur une zone de la couche conductrice électriquement destinée à former une zone de contact électrique, formant ainsi la deuxième ouverture 42. La seconde couche 6 est réalisée d'une part par reprise de croissance épitaxiale à partir de l'électrode 3 et de la première couche 5, et d'autre part par croissance épitaxiale à partir de nanoparticules de diamant déposée sur la couche conductrice électriquement 2. Il est à noter que les nanoparticules de diamant pour la formation de la seconde couche 6 peuvent également être déposées sur la première couche 5 et l'électrode 3, lesdites nanoparticules de diamant n'influençant pas la reprise de croissance épitaxiale à partir de la première couche et de l'électrode 3. A l'issue de cette étape, l'implant 1 est formé sur le substrat S.

Comme illustré sur la **figure 8f****,** une couche de protection P est déposée sur l'implant 1. Cette couche de protection P permet de protéger l'implant 1 lors de l'élimination du substrat S. La couche de protection P peut par exemple être un film comprenant un adhésif ou une résine.

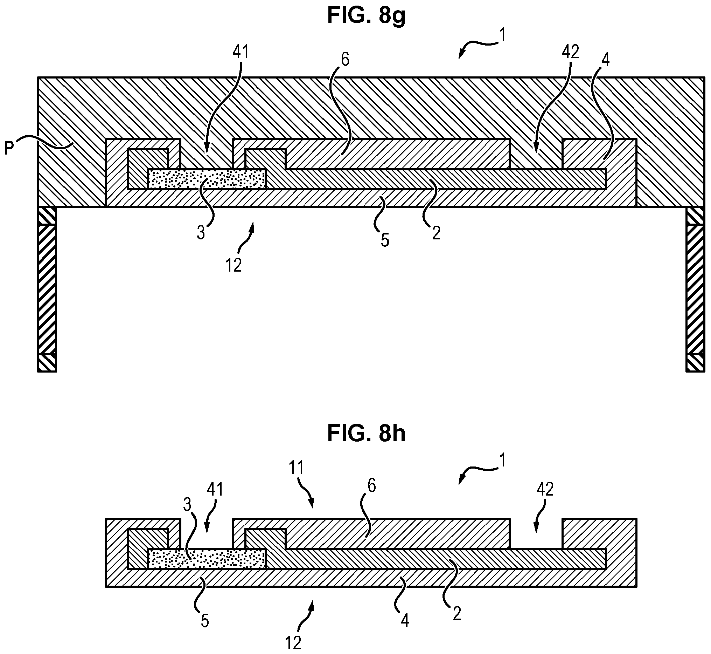

Comme illustré sur la **figure 8g****,** le substrat S est retiré, par exemple par gravure, libérant ainsi la face arrière 12 de l'implant 1.

Comme illustré sur la **figure 8h****,** la couche de protection P est ensuite retirée, libérant ainsi la face avant 11 de l'implant 1.

Lorsque la couche de protection P est un film avec un adhésif, la couche de protection P peut être retirée par une exposition à un rayonnement UV qui enlève son pouvoir adhérant à l'adhésif, puis en plaçant l'implant 1 dans une solution d'éthanol pour retirer le film.

Lorsque la couche de protection P est une résine, ladite couche de protection P peut être enlevée avec un solvant.

De plus, comme indiqué précédemment, l'implant 1 peut comprendre une couche en polymère afin de faciliter la manipulation dudit implant 1.

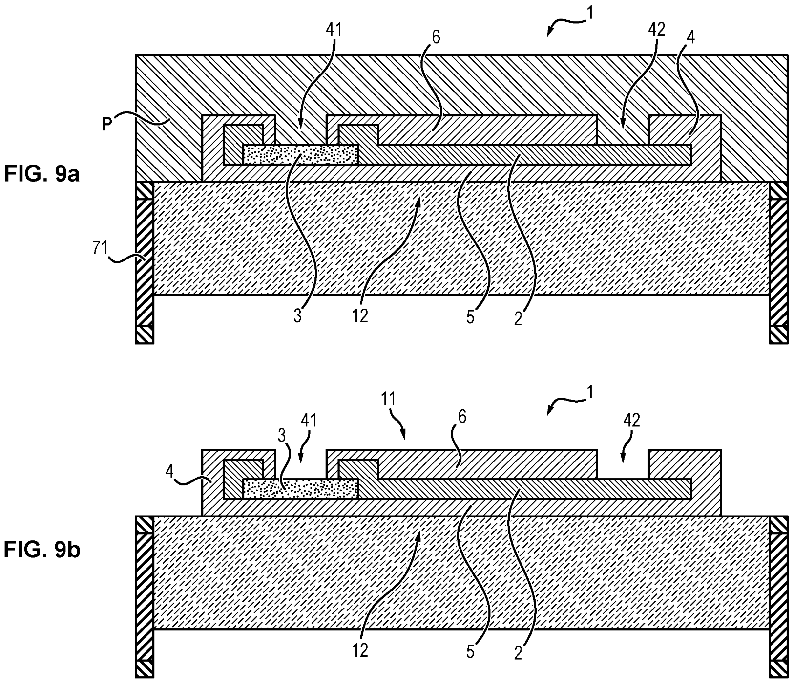

Comme illustré sur la **figure 9a****,** la première couche externe en polymère 71 peut être déposée sur la face arrière 12 de l'implant après le retrait du substrat S (par exemple après l'étape illustrée sur la figure 8g).

Comme illustré sur la **figure 9b****,** la couche de protection P est ensuite retirée de la face avant 11 de l'implant 1.

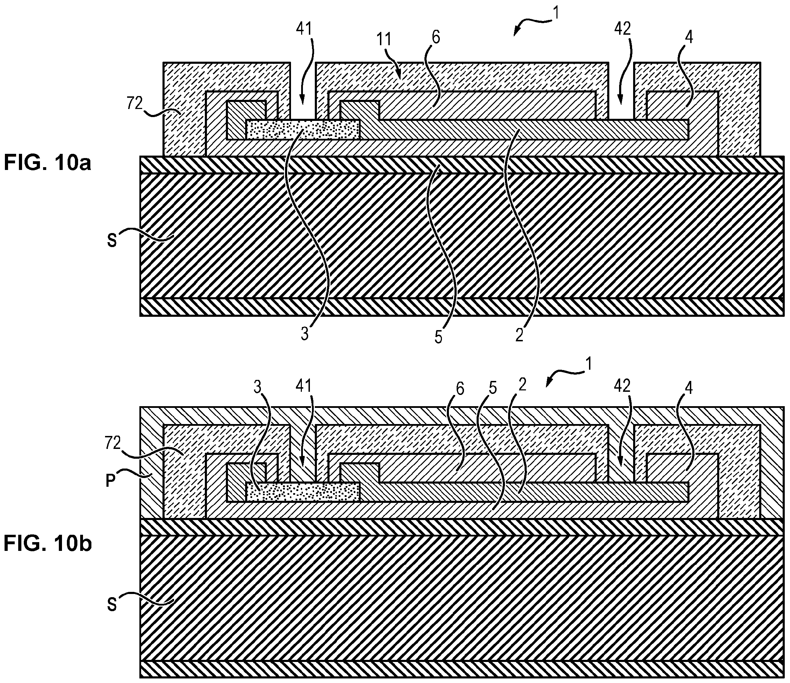

Comme illustré sur la **figure 10a****,** selon la variante dans laquelle l'implant 1 comprend la première couche externe en polymère 71 et la seconde couche externe en polymère 72, la seconde couche externe en polymère 72 est déposée sur la première face avant 11 de l'implant après le dépôt de la deuxième couche 6 en diamant isolant électriquement (par exemple après l'étape illustrée sur la figure 8e). La seconde couche externe en polymère 72 est gravée au niveau de la première ouverture 41 et la deuxième ouverture 42 pour laisser lesdites ouvertures en communication avec l'extérieur.

La couche de protection P est ensuite déposée sur la seconde couche externe de polymère 72, comme illustré sur la **figure 10b****.**

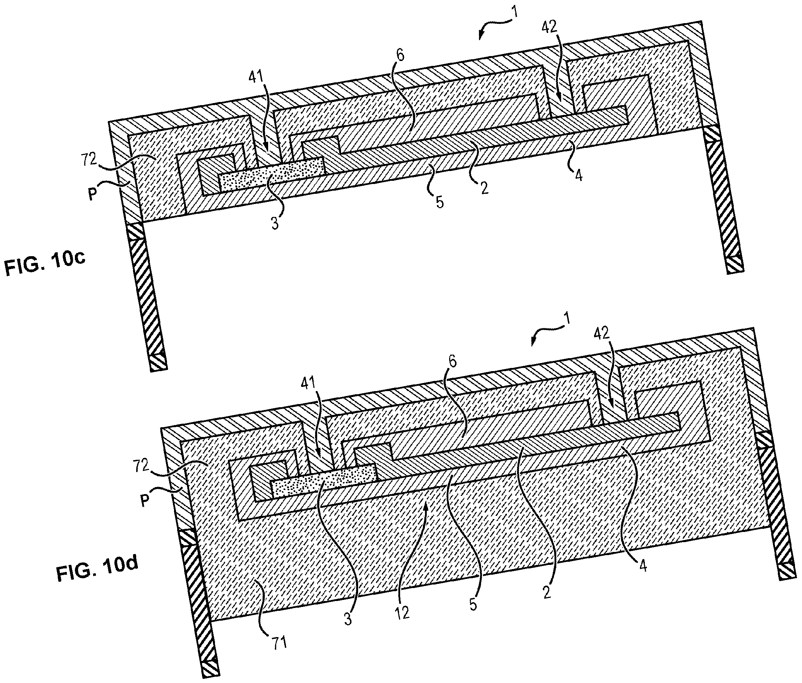

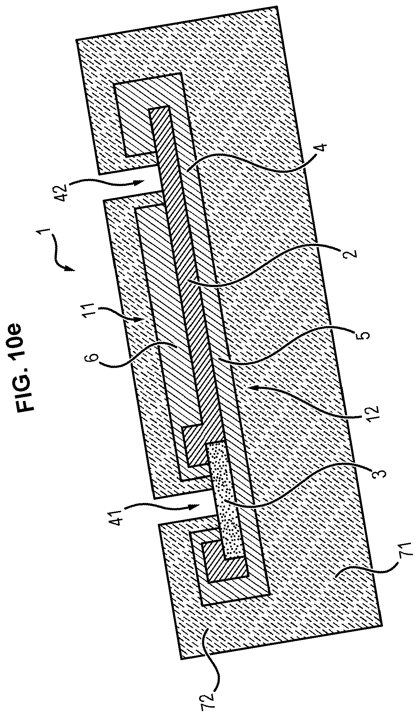

Ensuite, d'une manière similaire à la variante des **figures 9a** et **9b****,** et comme illustré aux **figures 10c, 10d** et **10e****,** le substrat S est retiré, la première couche externe en polymère 71 est déposée sur la face arrière 12 de l'implant souple 1, et la couche de protection P est retirée de la face avant 11 de l'implant souple 1.

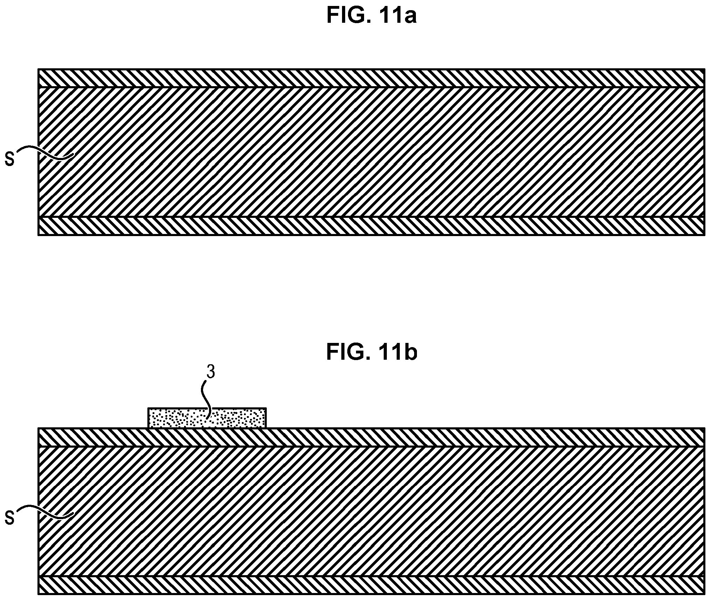

Une deuxième mise en œuvre possible du procédé de fabrication permettant de fabriquer un implant 1 selon le deuxième mode de réalisation est illustrée sur les **figures 11a -11h****.**

Comme visible sur la **figure 11a****,** un substrat S est fourni au début du procédé de fabrication afin de réaliser la succession d'étapes de dépôt des différentes couches formant l'implant souple 1.

Dans la deuxième mise en œuvre du procédé, comme illustrée sur la **figure 11b****,** la formation de l'électrode 3 en diamant dopé conducteur électriquement est réalisée en premier. L'électrode 3 est formée directement sur le substrat S. L'électrode 3 en diamant dopé conducteur électriquement est réalisée en déposant une couche de nanoparticules de diamant qui servent de point de démarrage pour la croissance épitaxiale du diamant. La croissance de l'électrode 3 en diamant dopé est ensuite réalisée par dépôt chimique en phase vapeur (ou CVD). La croissance pour le diamant étant faite à hautes températures, il est préférable que le substrat S soit en silicium, ou en quartz, plutôt qu'en polymère. Le diamant pour l'électrode 3 peut être au bore ou au phosphore.

Afin de déterminer la zone sur laquelle l'électrode 3 est formée, plusieurs solutions sont possibles. Il est par exemple possible de déposer les nanoparticules de diamant sur l'ensemble du substrat S, puis de retirer les nanoparticules de diamant par gravure dans les zones sur lesquelles il n'est pas souhaitée que l'électrode 3 soit formée, la zone d'intérêt étant quant à elle protégée de la gravure par un masque qui protège les nanoparticules de diamant sur cette zone d'intérêt. Il est également possible de déposer les nanoparticules de diamant uniquement sur la zone d'intérêt en recouvrant les zones sur lesquelles il n'est pas souhaité que l'électrode 3 soit formée par un masque lors du dépôt des nanoparticules de diamant.

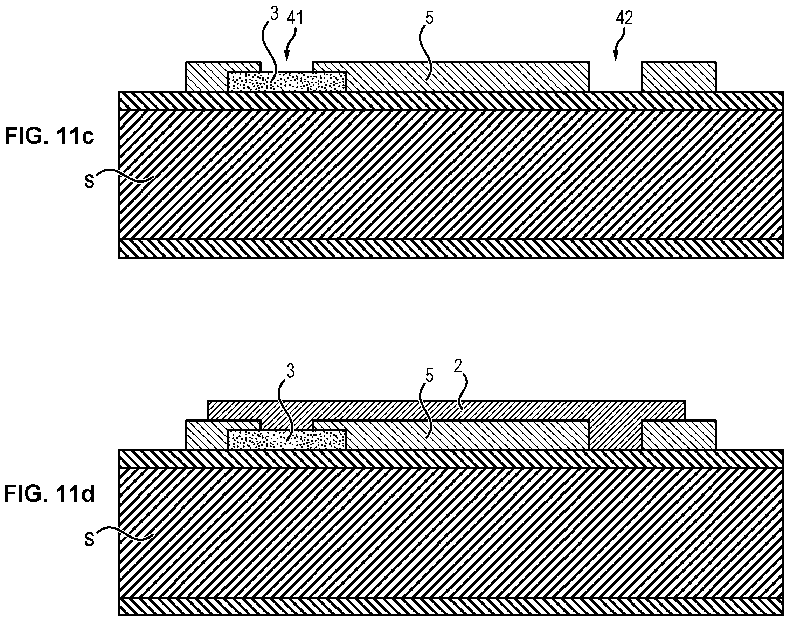

Ensuite, comme illustré sur la **figure 11c****,** la première couche 5 est formée par la création d'une couche en diamant isolant électriquement d'une part sur une partie du substrat et d'autre part sur le contour extérieur de l'électrode 3. La première couche 5 est formée par reprise de croissance épitaxiale à partir de l'électrode 3, réalisant ainsi une continuité de matière entre les deux couches en diamant. Le dépôt de la couche en diamant isolante électriquement pour former la première couche 5 est réalisée avec un masque afin que d'une part la première couche 5 ne recouvre pas l'électrode 3 sur une partie centrale de l'électrode 3, afin de permettre le contact entre l'électrode et la couche conductrice électriquement 2, et d'autre part afin de former la deuxième ouverture 42 pour la création de la zone définissant un contact électrique.

Comme illustrée par la **figure 11d****,** la couche conductrice électriquement 2 est ensuite formée sur la première couche 5. La couche conductrice électriquement 2 est en contact avec la partie centrale de l'électrode 3 non recouverte par la première couche 5. La couche conductrice électriquement 2 est également en contact avec le substrat S au niveau de la deuxième ouverture 42 formée dans la première couche 5, définissant ainsi un contact électrique. La couche conductrice électriquement 2 est formée par le dépôt d'une couche métallique, par exemple par dépôt chimique en phase vapeur. La couche conductrice électriquement 2 peut également être formée par le dépôt d'une couche en diamant dopé conducteur électriquement. La couche conductrice électriquement 2 est formée de manière à former une piste électrique pour l'électrode 3, et ainsi relier ladite électrode 3 à un composant électrique. Là encore, le contrôle de la forme de la couche conductrice électriquement 2 est réalisé par l'utilisation d'un masque.

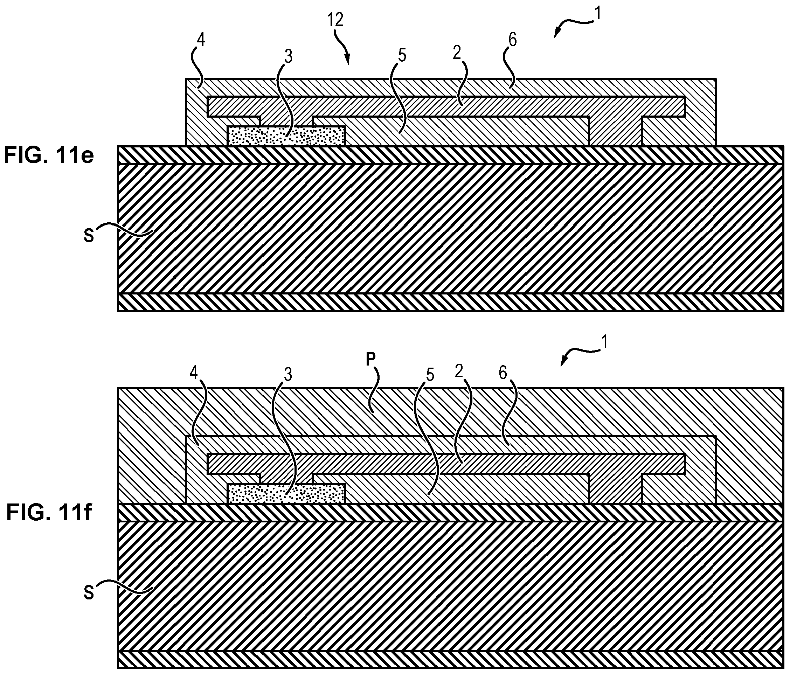

Comme illustré sur la **figure 11e****,** la seconde couche 6 en diamant isolant électriquement est ensuite formée par-dessus l'ensemble réalisé lors des étapes précédentes, finalisant ainsi la formation du revêtement de protection 4 autour de la couche conductrice électriquement 2. La seconde couche 6 recouvre intégralement la couche conductrice électriquement 2. La seconde couche 6 est réalisée d'une part par reprise de croissance épitaxiale à partie de la première couche 5, et d'autre part par croissance épitaxiale à partir de nanoparticules de diamant déposées sur la couche conductrice électriquement 2. A l'issue de cette étape, l'implant 1 est formé sur le substrat S, la face avant 11 de l'implant 1 étant contre le substrat S, et la face arrière 12 de l'implant 1 étant dirigée vers l'extérieur.

Comme illustré sur la **figure 11f****,** une couche de protection P est déposée sur la face arrière 12 l'implant 1. Cette couche de protection P permet de protéger l'implant 1 lors de l'élimination du substrat S. La couche de protection P peut exemple être un film comprenant un adhésif ou une résine.

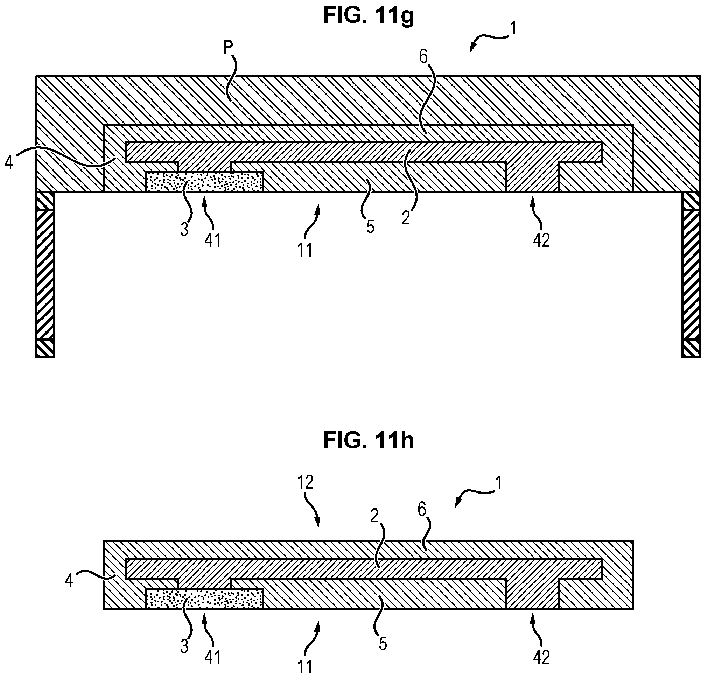

Comme illustré sur la **figure 11g****,** le substrat S est retiré, par exemple par gravure, libérant ainsi la face avant 11 de l'implant 1.

Comme illustré sur la **figure 11h****,** la couche de protection P est ensuite retirée, libérant ainsi la face arrière 12 de l'implant 1.

Lorsque la couche de protection P est un film avec un adhésif, la couche de protection P peut être retirée par une exposition à un rayonnement UV qui enlève son pouvoir adhérant à l'adhésif, puis en plaçant l'implant 1 dans une solution d'éthanol pour retirer le film.

Lorsque la couche de protection P est une résine, ladite couche de protection P peut être enlevée avec un solvant.

De plus, comme indiqué précédemment, l'implant 1 peut comprendre une couche en polymère afin de faciliter la manipulation dudit implant 1.

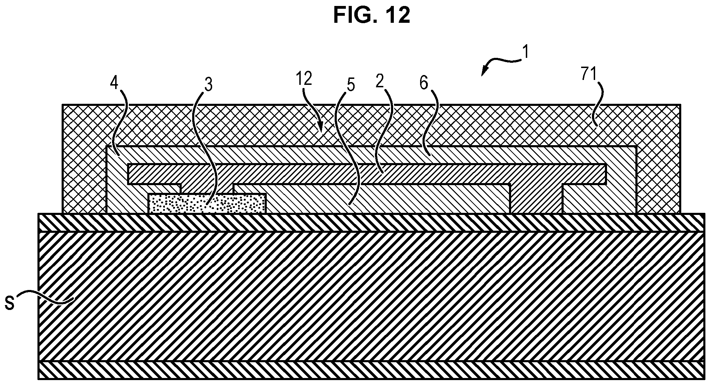

Comme illustré sur la **figure 12****,** la première couche externe en polymère 71 peut être déposée sur la face arrière 12 de l'implant avant le dépôt de la couche de protection P (par exemple après l'étape illustrée à la figure 11e).

Le substrat S et la couche de protection P sont ensuite retirés, comme cela est illustré sur la **figure 11g** et sur la **figure 11h****.**

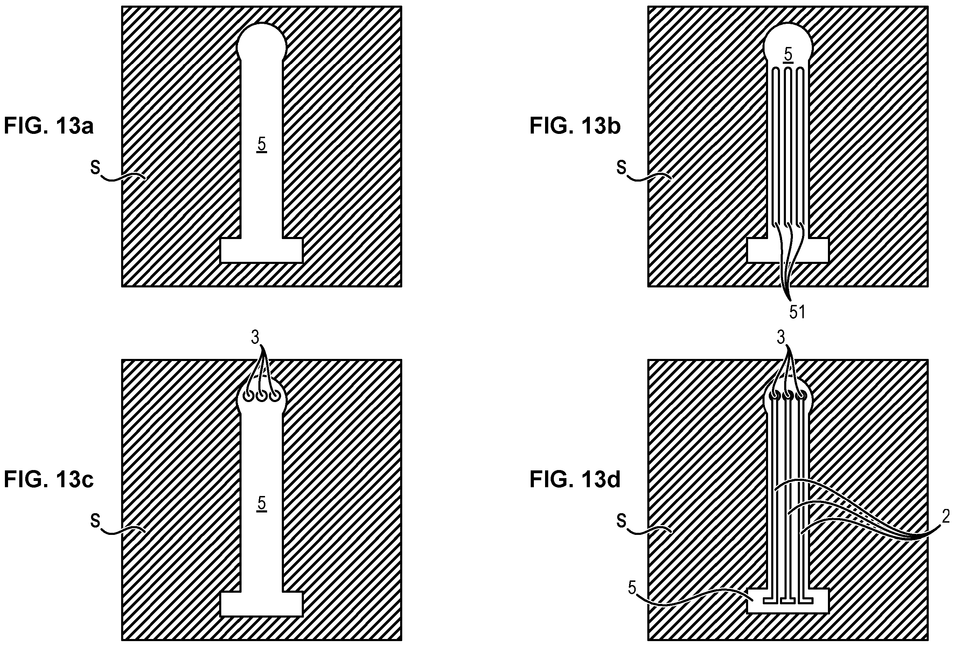

Les **figures 13a** - **13g** représentent les vues de dessus des différentes étapes de la première mise en œuvre du procédé de fabrication illustrées aux **figures 8a** - **8e** et **10a-10d.** la forme de l'implant 1 représentée dans ces figures n'est qu'un exemple possible de la forme qui peut être donnée à l'implant 1.

La **figure 13a** correspond à la première couche 5 en diamant isolant électriquement déposée sur le substrat, et est donc une vue de dessus de la **figure** 8a.

Ici cette première couche 5 en diamant isolant présente une épaisseur et une forme voulue de surface perpendiculaire à l'épaisseur.

La **figure 13b** correspond à une variante de la **figure 13a****,** dans laquelle la première couche 5 comprend des rainures 51 afin d'augmenter la souplesse de l'implant 1.

La **figure 13c** correspond à l'étape dans laquelle l'électrode 3 est formée sur la première couche 5 par reprise de croissance épitaxiale. Dans la variante représentée sur la figure 13c, trois électrodes 3 sont formées.

La **figure 13d** correspond à l'étape dans laquelle la couche conductrice électriquement 2 est formée sur la première couche 5 et sur l'électrode 3. L'implant 1 comprenant trois électrodes 3, la couche conductrice électriquement 2 forme trois pistes électriques, une piste pour chaque électrode 3.

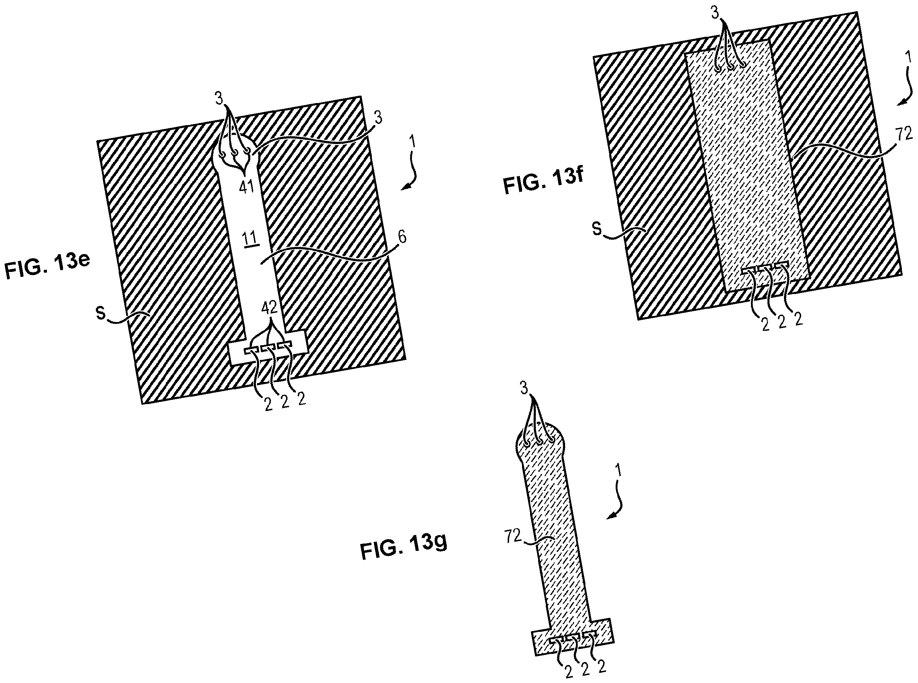

La **figure 13e** correspond à l'étape durant laquelle la deuxième couche 6 en diamant isolant électriquement est formée par reprise de croissance épitaxiale à partir de l'électrode 3 et de la première couche 5, de sorte à encapsuler de manière étanche la couche conductrice électriquement 2. Les électrodes 3 sont visibles au travers des premières ouvertures 41, une première ouverture 41 pour chaque électrode 3. Chaque piste formée par la couche conductrice électriquement 2 est visible au travers des deuxièmes ouvertures 42, une deuxième ouverture 42 pour chaque piste.

De même, la deuxième couche 6 en diamant isolant présente une épaisseur et une forme voulue de surface perpendiculaire à l'épaisseur.

La **figure 13f** correspond à l'étape dans laquelle la face avant 11 de l'implant 1 est recouverte par la seconde couche externe en polymère 72. Les électrodes 3 et la couche conductrice électriquement 2 sont accessibles au travers des ouvertures formées dans la seconde couche externe en polymère 72.

La **figure 13g** correspond à l'étape dans laquelle, le substrat S et la couche de protection P ont été retirées, séparant ainsi l'implant 1 de la galette (ou wafer selon la terminologie anglo-saxonne). De même que les premières et deuxièmes couches de diamant 5 et 6, la seconde couche externe en polymère 72 est découpée pour donner la forme finale à l'implant.

Comme visible sur les **figures 14****,** l'implant souple 1 selon l'invention possède une souplesse lui permettant d'épouser la forme de la surface sur laquelle il est déposé, même si la surface est très accidentée. Les **figures 14** montrent l'implant souple 1 situé sur une main dans un gant en latex G simulant la surface d'une structure nerveuse. La surface du gant comprend des bourrelets/plis B, et la forme de l'implant 1 épouse la forme des bourrelets/plis B des doigts et du dos de la main. L'implant souple 1 redéposé ensuite sur une table peut reprendre sa forme plane sur la surface plane de la table.

La souplesse de l'implant selon l'invention est comparable avec celle des implants de l'état de l'art qui ne comprennent pas des couches en diamant intrinsèque d'encapsulation de la piste conductrice.

Ainsi, l'épaisseur totale et la géométrie de l'implant 1 sont adaptées pour que l'implant souple 1 épouse la surface de l'organe d'intérêt, tel que le cortex cortical, le cœur, ou un muscle, l'épaisseur totale de l'implant étant inférieure à 20 µm.

L'épaisseur totale est égale à = épaisseur de la couche électrique 2+ épaisseur des couches en diamant isolant électriquement 5 et 6 + épaisseur couche externe polymère 72+ épaisseur couche externe polymère 71.

L'épaisseur totale est tellement petite que l'implant bien que comprenant du diamant (pour assurer une grande étanchéité et donc une grand robustesse de l'implant dans le temps) est extrêmement souple et peut être déformé comme souhaité pour prendre la forme de l'élément sur lequel on le pose.

## Revendications

1. Implant souple (1) pour la stimulation focale ou l'enregistrement électrique d'une structure nerveuse d'un organe d'intérêt, comprenant :
- au moins une couche externe en polymère biocompatible,
- une première couche (5) en diamant isolant électriquement,
- au moins une électrode (3) en diamant dopé conducteur électriquement en contact avec la première couche (5) en diamant isolant électriquement, située à une première ouverture (41),
- une couche conductrice électriquement (2) en métal ou en diamant dopé conducteur électriquement, en contact avec :
- l'électrode (3), et
- la première couche (5),
pour définir une piste conductrice par électrode (3) ;
- une seconde couche (6) en diamant isolant électriquement au moins en contact avec la couche conductrice électriquement (2) et une partie restante de la première couche (5),
agencés de façon à ce que :
- une étanchéité diamant isolant électriquement/diamant dopé conducteur électriquement est réalisée au niveau de l'électrode (3) par reprise de croissance épitaxiale ;
- la couche conductrice électriquement (2) est encapsulée par :
• l'électrode (3), la première couche (5), et la seconde couche (6), au niveau de l'électrode (3), et
• la première couche (5), et la seconde couche (6), sur toute sa surface restante, sauf sur une zone située à une seconde ouverture (42) définissant un contact électrique par électrode (3) pour connecter l'implant souple (1) via la couche conductrice électriquement (2) à un composant électronique afin de contrôler le couplage électrique entre l'électrode (3) et la structure nerveuse,
l'implant souple (1) disposant de deux faces :
- une face avant (11) comportant l'une des deux couches en diamant isolant électriquement localement ouverte, permettant un accès à l'électrode (3) et à la zone définissant un contact électrique pour l'électrode (3) ;
- une face arrière (12) comportant l'autre des deux couches en diamant isolant électriquement,
la première couche (5), l'électrode (3), et la seconde couche (6) présentent chacune des épaisseurs inférieures à un micromètre, avantageusement 500 nm,
la couche conductrice électriquement (2) présente une épaisseur inférieure à 3 µm, l'épaisseur totale et la géométrie de l'implant souple (1) étant adaptées pour que l'implant souple (1) épouse la surface de l'organe d'intérêt, l'épaisseur totale de l'implant souple (1) étant inférieure à 20 µm de façon à lui conférer de la souplesse.

2. Implant souple (1) selon la revendication 1, **caractérisé en ce que**, au niveau de l'électrode (3), la couche conductrice électriquement (2) est encapsulée par l'électrode (3) et la seconde couche (6) réalisée par reprise de croissance épitaxiale à partir de l'électrode (3),
l'électrode (3) étant encapsulée, sauf sur une partie centrale de mesure, par la première couche (5), par la deuxième couche (6) et par la couche conductrice électriquement (2).

3. Implant souple (1) selon la revendication 1, **caractérisé en ce que** la couche conductrice électriquement (2) est encapsulée par l'électrode (3) et les première et seconde couches (5, 6), la première couche (5) étant réalisée par reprise de croissance épitaxiale à partir de l'électrode (3).

4. Implant souple (1) selon l'une des revendications précédentes 1 à 3, **caractérisé en ce que** l'implant comporte une première couche externe en polymère (71) sur la face arrière (12) de l'implant (1).

5. Implant souple (1) selon la revendication précédente 4, **caractérisé en ce que** l'implant (1) comporte une seconde couche externe en polymère (72) sur la face avant (11) de l'implant (1) sauf au niveau d'au moins une partie de mesure de l'électrode (3) et au niveau d'une zone de contact électrique,
l'implant (1) étant ainsi complètement encapsulé par du polymère, sauf au niveau de la partie de mesure de l'électrode (3) et au niveau de la zone de contact électrique.

6. Implant souple (1) selon l'une des revendications précédentes 3 à 5, **caractérisé en ce que** la (ou les) couche(s) externe(s) en polymère présente(nt) une épaisseur comprise entre 2 et 10 µm.

7. Implant souple (1) selon l'une des revendications précédentes 3 à 6, dans lequel le polymère est un polymère biodégradable.

8. Implant souple (1) selon l'une des revendications précédentes 1 à 2, 4 à 7, dans lequel l'électrode en diamant dopé est nanostructurée.

9. Implant souple (1) selon l'une des revendications précédentes 1 à 8, dans lequel l'épaisseur totale et la géométrie de l'implant souple (1) étant adaptées pour que l'implant souple (1) épouse la surface de l'organe d'intérêt, l'épaisseur totale de l'implant souple (1) étant inférieure à 10 µm de façon à lui conférer de la souplesse.

10. Implant souple (1) selon l'une des revendications précédentes, dans lequel le diamant des éléments suivants : électrode (3), première couche (5), seconde couche (6), et couche conductrice électriquement (2) quand elle est en diamant,
n'est pas ultra-nanocristallin (UNCD).

11. Implant souple (1) selon l'une des revendications précédentes, dans lequel le diamant pour l'électrode (3) est dopé au bore ou au phosphore.

12. Implant souple (1) selon l'une des revendications précédentes, dans lequel le rapport de la longueur entre la première ouverture (41) et la seconde ouverture (42), sur l'épaisseur de l'implant, est supérieur à 100.

13. Procédé de fabrication d'au moins un implant souple (1) pour la stimulation focale ou l'enregistrement électrique d'une structure nerveuse d'un organe d'intérêt, défini selon l'une des revendications précédentes, ledit implant (1) étant réalisé par les étapes suivantes à partir d'un substrat (S) :
- (E1) réalisation d'une première couche (5) en diamant isolant électriquement et d'au moins une électrode (3) en diamant dopé conducteur électriquement par croissance épitaxiale, l'électrode (3) et la première couche (5) étant déposées l'une sur l'autre par reprise de croissance épitaxiale à l'issue de cette étape (E1) ;
- (E2) dépôt d'une couche conductrice électriquement (2) en appui sur au moins une partie de l'électrode (3) et au moins une partie donnée de la première couche (5), pour définir une piste conductrice par électrode (3) ;
- (E3) réalisation d'une seconde couche (6) en appui sur au moins une partie de la couche conductrice électriquement (2), et au moins sur une partie restante de la première couche (5), par croissance épitaxiale ;
les étapes de procédé sont réalisées pour que :
- une étanchéité diamant isolant électriquement/diamant dopé conducteur électriquement est réalisée au niveau de l'électrode (3) par reprise de croissance épitaxiale,
- la couche conductrice électriquement (2) est encapsulée par :
l'électrode (3), la première couche (5), et la seconde couche (6), au niveau de l'électrode (3) et
la première couche (5), et la seconde couche (6), sur toute sa surface restante sauf sur une zone définissant un contact électrique par électrode (3).

14. Procédé selon la revendication 13, dans lequel l'épaisseur totale de l'implant souple est inférieure à 20 µm.

15. Procédé selon l'une des revendications précédentes 13 à 14, dans lequel:
- dans l'étape (E1), réalisation par croissance épitaxiale de la première couche (5) en diamant isolant électriquement sur le substrat (S), puis réalisation par reprise de croissance épitaxiale d'au moins une électrode (3) en diamant dopé conducteur électriquement en appui sur la première couche (5),
- dans l'étape (E2), dépôt de la couche conductrice électriquement (2) sur un contour extérieur de l'électrode (3) et au moins sur une partie donnée de la première couche (5),
- dans l'étape (E3), la croissance épitaxiale de la deuxième couche (6) est réalisée sur :
la couche conductrice électriquement (2) sauf sur une zone définissant un contact électrique par piste conductrice,
ainsi que sur l'électrode (3) par reprise de croissance épitaxiale, en laissant à l'air libre une partie centrale de mesure de l'électrode (3),
cette reprise de croissance diamant isolant électriquement sur diamant dopé conducteur électriquement permet de garantir l'étanchéité de l'implant au niveau de l'électrode (3).

16. Procédé selon l'une des revendications précédentes 13 à 14, dans lequel :
- dans l'étape (E1), réalisation par croissance épitaxiale d'au moins une électrode (3) en diamant dopé conducteur électriquement sur le substrat (S),
puis réalisation de la première couche (5) en diamant sur le conteur extérieur de l'électrode (3) par reprise de croissance épitaxiale et sur une partie du substrat (S) par croissance épitaxiale sauf dans une zone donnée qui définit un contact électrique,
- dans l'étape (E2), dépôt de la couche conductrice (2) sur au moins une partie centrale de l'électrode (3), sur au moins une partie donnée de la première couche (5) pour réaliser une piste conductrice par électrode (3), et sur la zone donnée de contact électrique pour réaliser un contact électrique par piste conductrice.

17. Procédé selon l'une des revendications 13 à 16, comprenant l'étape supplémentaire suivante (E4) de dépôt d'au moins une couche externe en polymère, par exemple sur la face arrière (12) de l'implant (1).

18. Procédé selon la revendication précédente 17, comprenant les étapes supplémentaires suivantes :
- (E41) dépôt d'une couche externe en polymère sur la face avant de l'implant, où se situent l'électrode (3) et le contact électrique associé, ainsi que sur le substrat (S) ;
- (E42) ouverture de la couche externe en polymère au niveau du contact électrique et de l'électrode (3) et dépôt d'une résine de protection ;
- (E43) gravure du substrat jusqu'à découvrir la face arrière (12) de l'implant ;
- (E44) dépôt d'une autre couche externe en polymère sur la face arrière de l'implant élimination de la résine de protection et découpe de l'implant pour lui donner sa forme finale.

19. Procédé selon l'une des revendications précédentes 14-15,17-18, dans lequel l'électrode (3) est nanostructurée de la façon suivante :
- dépôt sur l'électrode (3) d'un matériau formant un motif 3D nanostructuré ;
- dépôt d'une couche en diamant dopé conducteur électriquement par-dessus le matériau formant le motif nanostructuré.

## Patentansprüche

1. Flexibles Implantat (1) zur fokalen Stimulation oder elektrischen Aufzeichnung einer Nervenstruktur eines interessierenden Organs, das Folgendes aufweist:
- mindestens eine äußere Schicht aus biokompatiblem Polymer,
- eine erste Schicht (5) aus elektrisch isolierendem Diamant,
- mindestens eine Elektrode (3) aus dotiertem, elektrisch leitfähigem Diamant in Kontakt mit der ersten Schicht (5) aus elektrisch isolierendem Diamant, die sich an einer ersten Öffnung (41) befindet,
- eine elektrisch leitfähige Schicht (2) aus Metall oder aus dotiertem, elektrisch leitfähigem Diamant, die in Kontakt ist mit:
- der Elektrode (3), und
- der ersten Schicht (5),
um eine Leiterbahn pro Elektrode (3) zu definieren;
- eine zweite Schicht (6) aus elektrisch isolierendem Diamant, die mindestens in Kontakt mit der elektrisch leitfähigen Schicht (2) und einem restlichen Teil der ersten Schicht (5) ist,
die so angeordnet sind, dass:
- eine Dichtheit zwischen elektrisch isolierendem Diamant/dotiertem, elektrisch leitfähigem Diamant
auf Höhe der Elektrode (3) durch erneutes epitaktisches Wachstum erzeugt wird;
- die elektrisch leitfähige Schicht (2) eingekapselt ist durch:
• die Elektrode (3), die erste Schicht (5) und die zweite Schicht (6) an der Elektrode (3), und
• die erste Schicht (5) und die zweite Schicht (6) über ihre gesamte verbleibende Oberfläche, außer an einem Bereich, der sich an einer zweiten Öffnung (42) befindet, die einen elektrischen Kontakt pro Elektrode (3) definiert, um das flexible Implantat (1) über die elektrisch leitfähige Schicht (2) mit einer elektronischen Komponente zu verbinden, um die elektrische Kopplung zwischen der Elektrode (3) und der Nervenstruktur zu steuern,
das flexible Implantat (1), das über zwei Seiten verfügt:
- eine Vorderseite (11) mit einer der beiden lokal offenen Schichten aus elektrisch isolierendem Diamant, die den Zugang zur Elektrode (3) und zu dem Bereich, der einen elektrischen Kontakt für die Elektrode (3) definiert, ermöglicht;
- eine Rückseite (12), die die andere der beiden Schichten aus elektrisch isolierendem Diamant umfasst,
die erste Schicht (5), die Elektrode (3) und die zweite Schicht (6) weisen jeweils eine Dicke von weniger als einem Mikrometer, vorteilhafterweise von 500 nm auf,
die elektrisch leitfähige Schicht (2) weist eine Dicke von weniger als 3 µm auf, wobei die Gesamtdicke und die Geometrie des flexiblen Implantats (1) so ausgebildet sind, dass sich das flexible Implantat (1) an die Oberfläche des interessierenden Organs anpasst, wobei die Gesamtdicke des flexiblen Implantats (1) weniger als 20 µm beträgt, um ihm Flexibilität zu verleihen.

2. Flexibles Implantat (1) nach Anspruch 1, **dadurch gekennzeichnet, dass** an der Elektrode (3) die elektrisch leitfähige Schicht (2) von der Elektrode (3) eingekapselt ist und die zweite Schicht (6) durch erneutes epitaktisches Wachstum aus der Elektrode (3) erzeugt wird,
wobei die Elektrode (3), mit Ausnahme eines zentralen Teils zur Messung, von der ersten Schicht (5), von der zweiten Schicht (6) und von der elektrisch leitfähigen Schicht (2) eingekapselt ist.

3. Flexibles Implantat (1) nach Anspruch 1, **dadurch gekennzeichnet, dass** die elektrisch leitfähige Schicht (2) von der Elektrode (3) und der ersten und zweiten Schicht (5, 6) eingekapselt ist, wobei die erste Schicht (5) durch erneutes epitaktisches Wachstum aus der Elektrode (3) erzeugt wird.

4. Flexibles Implantat (1) nach einem der vorhergehenden Ansprüche 1 bis 3, **dadurch gekennzeichnet, dass** das Implantat eine erste äußere Schicht aus Polymer (71) an der Rückseite (12) des Implantats (1) aufweist.

5. Flexibles Implantat (1) nach dem vorhergehenden Anspruch 4, **dadurch gekennzeichnet, dass** das Implantat (1) eine zweite äußere Schicht aus Polymer (72) an der Vorderseite (11) des Implantats (1) mit Ausnahme an mindestens einem Messbereich der Elektrode (3) und an einer elektrischen Kontaktzone aufweist,
womit das Implantat (1), mit Ausnahme am Messbereich der Elektrode (3) und an der elektrischen Kontaktzone, vollständig eingekapselt ist.

6. Flexibles Implantat (1) nach einem der vorhergehenden Ansprüche 3 bis 5, **dadurch gekennzeichnet, dass** die äußere(n) Schicht(en) aus Polymer eine Dicke zwischen 2 und 10 µm aufweist (aufweisen).

7. Flexibles Implantat (1) nach einem der vorhergehenden Ansprüche 3 bis 6, bei dem das Polymer ein biologisch abbaubares Polymer ist.

8. Flexibles Implantat (1) nach einem der vorhergehenden Ansprüche 1 bis 2, 4 bis 7, bei dem die Elektrode aus dotiertem Diamant nanostrukturiert ist.

9. Flexibles Implantat (1) nach einem der vorhergehenden Ansprüche 1 bis 8, bei dem die Gesamtdicke und die Geometrie des flexiblen Implantats (1) so ausgebildet sind, dass sich das flexible Implantat (1) an die Oberfläche des interessierenden Organs anpasst, wobei die Gesamtdicke des flexiblen Implantats (1) weniger als 10 µm beträgt, um ihm Flexibilität zu verleihen.

10. Flexibles Implantat (1) nach einem der vorhergehenden Ansprüche, bei dem der Diamant der folgenden Elemente: Elektrode (3), erste Schicht (5), zweite Schicht (6) und elektrisch leitfähige Schicht (2), wenn sie aus Diamant ist, nicht ultrananokristallin (UNCD) ist.

11. Flexibles Implantat (1) nach einem der vorhergehenden Ansprüche, bei dem der Diamant für die Elektrode (3) mit Bor oder Phosphor dotiert ist.

12. Flexibles Implantat (1) nach einem der vorhergehenden Ansprüche, bei dem das Verhältnis der Länge zwischen der ersten Öffnung (41) und der zweiten Öffnung (42) zur Dicke des Implantats größer als 100 ist.

13. Verfahren zur Herstellung mindestens eines flexiblen Implantats (1) zur fokalen Stimulation oder elektrischen Aufzeichnung einer Nervenstruktur eines interessierenden Organs, definiert nach einem der vorhergehenden Ansprüche, wobei das genannte Implantat (1) durch die folgenden Schritte aus einem Substrat (S) hergestellt wird:
- (E1) Erzeugung einer ersten Schicht (5) aus elektrisch isolierendem Diamant und mindestens einer Elektrode (3) aus dotiertem, elektrisch leitfähigem Diamant durch epitaktisches Wachstum, wobei die Elektrode (3) und die erste Schicht (5) am Ende dieses Schrittes (E1) durch erneutes epitaktisches Wachstum übereinander aufgebracht sind;
- (E2) Aufbringen einer elektrisch leitfähigen Schicht (2), die auf mindestens einem Bereich der Elektrode (3) und mindestens einem bestimmten Bereich der ersten Schicht (5) aufliegt, um eine Leiterbahn pro Elektrode (3) zu definieren;
- (E3) Erzeugung, durch epitaktisches Wachstum, einer zweiten Schicht (6), die auf mindestens einem Bereich der elektrisch leitfähigen Schicht (2) und mindestens einem restlichen Bereich der ersten Schicht (5) aufliegt;
die Verfahrensschritte so ausgeführt werden, dass:
- eine Dichtheit zwischen elektrisch isolierendem Diamant/dotiertem, elektrisch leitfähigem Diamant auf Höhe der Elektrode (3) durch erneutes epitaktisches Wachstum erzeugt wird,
- die elektrisch leitfähige Schicht (2) eingekapselt wird durch:
die Elektrode (3), die erste Schicht (5) und die zweite Schicht (6) an der Elektrode (3) und
die erste Schicht (5), und die zweite Schicht (6) über ihre gesamte verbleibende Oberfläche, außer an einem Bereich, der einen elektrischen Kontakt pro Elektrode (3) definiert.

14. Verfahren nach Anspruch 13, bei dem die Gesamtdicke des flexiblen Implantats weniger als 20 µm beträgt.

15. Verfahren nach einem der vorhergehenden Ansprüche 13 bis 14, mit:
- bei Schritt (E1) der Erzeugung durch epitaktisches Wachstum der ersten Schicht (5) aus elektrisch isolierendem Diamant auf dem Substrat (S) und dann der Erzeugung durch erneutes epitaktisches Wachstum von mindestens einer Elektrode (3) aus dotiertem, elektrisch leitfähigem Diamant, die auf der ersten Schicht (5) aufliegt,
- bei Schritt (E2) dem Aufbringen der elektrisch leitfähigen Schicht (2) auf einer Außenkontur der Elektrode (3) und mindestens auf einem bestimmten Bereich der ersten Schicht (5),
- bei Schritt (E3) dem epitaktischen Wachstum der zweiten Schicht (6) auf:
der elektrisch leitfähigen Schicht (2), außer an einem Bereich, der einen elektrischen Kontakt pro Leiterbahn definiert, sowie auf der Elektrode (3) durch erneutes epitaktisches Wachstum,
wobei ein zentraler Messbereich der Elektrode (3) freigelassen wird und dieses erneute Wachstum von elektrisch isolierendem Diamant auf dotiertem, elektrisch leitfähigem Diamant die Dichtheit des Implantats auf Höhe der Elektrode (3) gewährleistet.

16. Verfahren nach einem der vorhergehenden Ansprüche 13 bis 14, mit:
- bei Schritt (E1) der Erzeugung durch epitaktisches Wachstum von mindestens einer Elektrode (3) aus dotiertem, elektrisch leitfähigem Diamant auf dem Substrat (S), dann der Erzeugung der ersten Schicht (5) aus Diamant auf der Außenkontur der Elektrode (3) durch erneutes epitaktisches Wachstum mit Ausnahme eines bestimmten Bereichs, der einen elektrischen Kontakt definiert,
- bei Schritt (E2) dem Aufbringen der elektrisch leitfähigen Schicht (2) auf mindestens einem zentralen Bereich der Elektrode (3), auf mindestens einem bestimmten Bereich der ersten Schicht (5), um eine Leiterbahn pro Elektrode (3) zu erzeugen, und auf dem gegebenen elektrischen Kontaktbereich, um einen elektrischen Kontakt pro Leiterbahn herzustellen.

17. Verfahren nach einem der Ansprüche 13 bis 16, das den folgenden zusätzlichen Schritt (E4) umfasst, bei dem mindestens eine äußere Schicht aus Polymer, beispielsweise auf der Rückseite (12) des Implantats (1) aufgebracht wird.

18. Verfahren nach dem vorhergehenden Anspruch 17, das die folgenden zusätzlichen Schritte umfasst:
- (E41) Aufbringen einer äußeren Schicht aus Polymer auf der Vorderseite des Implantats, auf der sich die Elektrode (3) und der dazugehörige elektrische Kontakt befinden, sowie auf dem Substrat (S);
- (E42) Öffnung der äußeren Schicht aus Polymer auf Höhe des elektrischen Kontakts und der Elektrode (3) und Aufbringen eines Schutzharzes;
- (E43) Ätzen des Substrats zum Freilegen der Rückseite (12) des Implantats;
- (E44) Aufbringen einer weiteren äußeren Schicht aus Polymer auf der Rückseite des Implantats
Entfernen des Schutzharzes und Schneiden des Implantats, um ihm seine endgültige Form zu geben.

19. Verfahren nach einem der vorhergehenden Ansprüche 14-15,17-18, bei dem die Elektrode (3) folgendermaßen nanostrukturiert ist:
- Aufbringen auf der Elektrode (3) eines Materials, das ein nanostrukturiertes 3D-Muster bildet;
- Aufbringen einer Schicht aus dotiertem, elektrische leitfähigem Diamant auf dem Material, welches das nanostrukturierte Muster bildet.

## Claims

1. Flexible implant (1) for focal stimulation or electrical recording of a nerve structure of an organ of interest, comprising :
- at least one outer layer of biocompatible polymer,
- a first layer (5) of electrically insulating diamond,
- at least one electrode (3) of electrically conductive doped diamond in contact with the first layer (5) of electrically insulating diamond, located at a first opening (41),
- an electrically conductive layer (2) of metal or electrically conductive doped diamond, in contact with :
- the electrode (3), and
- the first layer (5),
to define a conductive track per electrode (3) ;
- a second layer (6) of electrically insulating diamond at least in contact with the electrically conductive layer (2) and a remaining part of the first layer (5),
arranged so that:
- an electrically insulating diamond/electrically conductive doped diamond seal is made at the electrode (3) by epitaxial growth ;
- the electrically conductive layer (2) is encapsulated by :
• the electrode (3), the first layer (5) and the second layer (6) at the electrode (3), and
• the first layer (5), and the second layer (6), over its entire remaining surface, except for an area located at a second opening (42) defining an electrical contact by electrode (3) for connecting the flexible implant (1) via the electrically conductive layer (2) to an electronic component to control the electrical coupling between the electrode (3) and the nerve structure,
the flexible implant (1) with two faces:
- a front face (11) having one of the two electrically insulating diamond layers locally open, allowing access to the electrode (3) and to the area defining an electrical contact for the electrode (3) ;
- a back face (12) with the other of the two electrically insulating diamond layers,
the first layer (5), the electrode (3), and the second layer (6) each have thicknesses of less than one micrometer, preferably 500 nm,
the electrically conductive layer (2) has a thickness of less than 3 µm,
the total thickness and geometry of the flexible implant (1) being adapted so that the flexible implant (1) conforms to the surface of the organ of interest, the total thickness of the flexible implant (1) being less than 20 µm so as to provide flexibility.

2. Flexible implant (1) according to claim 1, wherein, at the electrode (3), the electrically conductive layer (2) is encapsulated by the electrode (3) and the second layer (6) is epitaxially grown from the electrode (3),
the electrode (3) is encapsulated, except for a central measuring part, by the first layer (5), the second layer (6) and the electrically conductive layer (2).

3. Flexible implant (1) according to claim 1, wherein the electrically conductive layer (2) is encapsulated by the electrode (3) and the first and second layers (5, 6), the first layer (5) being grown epitaxially from the electrode (3).

4. Flexible implant (1) according to any of the preceding claims 1 to 3, wherein the implant has a first outer polymer layer (71) on the back face (12) of the implant (1).

5. Flexible implant (1) according to the preceding claim 4, wherein the implant (1) comprises a second outer polymer layer (72) on the front face (11) of the implant (1) except at the level of at least one measuring portion of the electrode (3) and at the level of an electrical contact zone,
the implant (1) thus being completely encapsulated by polymer, except at the measuring portion of the electrode (3) and at the electrical contact area.

6. Flexible implant (1) according to any of the preceding claims 3 to 5, wherein the outer polymer layer(s) has/have a thickness of between 2 and 10 µm.

7. Flexible implant (1) according to any of the preceding claims 3 to 6, wherein the polymer is a biodegradable polymer.

8. Flexible implant (1) according to any of the preceding claims 1 to 2, 4 to 7, wherein the doped diamond electrode is nanostructured.

9. Flexible implant (1) according to any of the preceding claims 1 to 8, wherein the total thickness and geometry of the flexible implant (1) being adapted so that the flexible implant (1) conforms to the surface of the organ of interest, the total thickness of the flexible implant (1) being less than 10µm so as to impart flexibility.

10. Flexible implant (1) according to any of the preceding claims, wherein the diamond of the following elements: electrode (3), first layer (5), second layer (6), and electrically conductive layer (2) when made of diamond, is not ultra-nanocrystalline (UNCD).

11. Flexible implant (1) according to any of the preceding claims, wherein the diamond for the electrode (3) is doped with boron or phosphorus.

12. Flexible implant (1) according to any of the preceding claims, wherein the ratio of the length between the first opening (41) and the second opening (42) to the thickness of the implant is greater than 100.

13. Process of manufacturing at least one flexible implant (1) for the focal stimulation or electrical recording of a nerve structure of an organ of interest, defined according to one of the preceding claims, said implant (1) being made by the following steps from a substrate (S):
- (E1) manufacturing a first layer (5) of electrically insulating diamond and at least one electrode (3) of electrically conductive doped diamond by epitaxial growth, the electrode (3) and the first layer (5) being deposited one on top of the other by epitaxial growth at the end of this step (E1);
- (E2) deposition of an electrically conductive layer (2) abutting at least a portion of the electrode (3) and at least a given portion of the first layer (5), to define a conductive track per electrode (3);
- (E3) manufacturing a second layer (6) resting on at least part of the electrically conductive layer (2), and on at least a remaining part of the first layer (5), by epitaxial growth ;
the process steps are performed so that:
- an electrically insulating diamond/electrically conductive doped diamond seal is made at the electrode (3) by epitaxial growth,
- the electrically conductive layer (2) is encapsulated by :
the electrode (3), the first layer (5), and the second layer (6), at the electrode (3) and
the first layer (5), and the second layer (6), over its entire remaining surface except for an area defining an electrical contact per electrode (3).

14. Process of claim 13, wherein the total thickness of the flexible implant is less than 20 µm.

15. Process according to one of the preceding claims 13 to 14, wherein :
- in step (E1), epitaxial growth of the first layer (5) of electrically insulating diamond on the substrate (S), followed by epitaxial growth of at least one electrode (3) of electrically conductive doped diamond bearing on the first layer (5),
- in step (E2), deposition of the electrically conductive layer (2) on an outer contour of the electrode (3) and at least on a given part of the first layer (5),
- in step (E3), epitaxial growth of the second layer (6) is carried out on :
the electrically conductive layer (2) except on a zone defining an electrical contact by conductive track, as well as on the electrode (3) by epitaxial re-growth, leaving a central measuring portion of the electrode (3) in the open air, this re-growth of electrically insulating diamond on electrically conductive doped diamond makes it possible to guarantee the tightness of the implant at the electrode (3).

16. Process according to one of the preceding claims 13 to 14, in which:
- in step (E1), epitaxial growth of at least one electrically conductive doped diamond electrode (3) on the substrate (S),
then manufacturing the first diamond layer (5) on the outer contour of the electrode (3) by epitaxial growth and on a part of the substrate (S) by epitaxial growth except in a given area which defines an electrical contact,
- in step (E2), depositing the conductive layer (2) on at least a central portion of the electrode (3), on at least a given portion of the first layer (5) to make a conductive track per electrode (3), and on the given area of electrical contact to make an electrical contact per conductive track.

17. Process according to any of claims 13 to 16, comprising the following additional step (E4) for deposition at least one outer polymer layer, for example on the back face (12) of the implant (1).

18. Process according to the preceding claim 17, comprising the following additional steps:
- (E41) deposition an outer polymer layer on the front face of the implant, where the electrode (3) and the associated electrical contact are located, and on the substrate (S) ,
- (E42) opening the outer polymer layer at the electrical contact and the electrode (3) and deposition a protective resin;
- (E43) engraving the substrate until the back face (12) of the implant is exposed;
- (E44) deposition another outer polymer layer on the back face of the implant removing the protective resin and cutting the implant to its final shape.

19. Process according to any of the preceding claims 14-15, 17-18, wherein the electrode (3) is nanostructured as follows:
- deposition on the electrode (3) of a material forming a nanostructured 3D pattern ;
- deposition an electrically conductive doped diamond layer over the nanostructured pattern material.
